(19) Europäisches Patentamt
European Patent Office
Office européen des brevets

(11) EP 1 243 581 B1

(12) EUROPEAN PATENT SPECIFICATION

(45) Date of publication and mention of the grant of the patent:
**13.07.2005 Bulletin 2005/28**

(21) Application number: **01201057.5**

(22) Date of filing: **21.03.2001**

(51) Int Cl.[7]: **C07D 213/82**, C07D 213/78, C07D 213/80, C07D 405/06, C07D 409/06, C07D 401/06, A61K 31/444, A61P 9/10, A61P 17/00, A61P 5/50

(54) **Pyridinium compounds useful for the treatment of AGE-related diseases**

Pyridiniumverbindungen zur Behandlung von AGE-relatierten Krankheiten

Composés pyridinium utilisés pour le traitement de maladies relatifs à l'AGE

(84) Designated Contracting States:
**AT BE CH CY DE DK ES FI FR GB GR IE IT LI LU MC NL PT SE TR**

(43) Date of publication of application:
**25.09.2002 Bulletin 2002/39**

(73) Proprietor: **Torrent Pharmaceuticals Ltd**
**Ahmedabad 380 009, Gujarat State (IN)**

(72) Inventor: **Sankaranarayanan, Alangudi**
**Ahmedabad - 380 054 (IN)**

(74) Representative: **Schmitz, Jean-Marie et al**
**Dennemeyer & Associates S.A.**
**P.O. Box 1502**
**1015 Luxembourg (LU)**

(56) References cited:
**WO-A-01/25208**

- **PATENT ABSTRACTS OF JAPAN vol. 2000, no. 01, 31 January 2000 (2000-01-31) & JP 11 292881 A (SHIONOGI &CO LTD), 26 October 1999 (1999-10-26)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 243 581 B1

## Description

The present, invention relates to a pyridinium derivative according to claim 1, a process for the preparation of pyridinium salt derivatives according to claim 2, the use of a compound according toclaims 3, 10 and 11, a pharmaceutical composition according to claim 4, a method for the preparation of a parenteral formulation according to claim 8.

The present invention relates to a new class of compounds of pyridinium series and to their use in treatment of diabetes and related illnesses. More particularly the invention relates to compounds of this series, methods for their preparation, pharmaceutical composition containing these compounds and their use in the treatment of complications of diabetes mellitus. The compounds of this series exhibit AGE breaking activity, which is essential for the treatment of diabetic and aging-related complications including kidney disease, nerve damage, atherosclerosis, retinopathy and dermatological conditions. The invention also extends to the method of reversing the discoloration of teeth resulting from nonenzymatic browning in the oral cavity which comprises administration of an amount effective to reverse preformed advanced glycosylation crosslinks.

Maillard in 1912 found that reducing sugars, such as glucose and ribose react with proteins to form brown pigments. Further studies have shown that this is an irreversible non-enzymatic reaction, which occurs in several natural systems including stored foodstuff. Maillard reaction occurs in two stages, early and advanced. Initially, proteins react with glucose to form stable Amadori products, which subsequently crosslinks to form advanced glycation end products (AGE). In most cases, the formation of AGE also accompanies browning of the proteins and increase in the fluorescence.

In diabetes, where blood glucose level is significantly higher than normal, the reaction of glucose with several proteins such as haemoglobin, lens crystallin and collagen, gives rise to the formation of AGE, which in turn, is responsible for the complications associated with diabetes, such as nephropathy, microangiopathy, endothelial dysfunction and other organ dysfunctions. In addition, the activity of several growth factors, such as basic fibroblast growth factor, is also impaired. AGE products, unlike normal proteins in tissue, have a slower rate of turnover and replenishment. It has been reported that AGE products may in fact elicit a complex immunological reaction involving RAGE (Receptor for Advanced Glycation End Products) receptors and activation of several incompletely defined immunological processes. It has been documented that diabetes with evidence of microangiopathy and macroangiopathy also show evidence of oxidative stress, the mechanism of which has not been elucidated.

In vitro AGE formation can be studied in the laboratory by incubating reducing sugars, such as ribose or glucose with bovine serum albumin. AGE formation can be detected by increase in the fluorescence or increased cross reactivity with anti-AGE antibodies. The increase in fluorescence seems to precede formation of AGE specific antigenic epitopes. This increase in fluorescence is used to monitor the increased AGE formation in vitro (Brownlee M et al, Science 1986; 232:1629-1632). In addition to the increase in the fluorescence, one of the most important features of in vitro AGE formation is the formation of antigenic epitopes that are specific to AGE and not to the native proteins. Therefore, it is possible to raise antibodies against advanced glycation end products of one protein and use them to detect AGE formation in other proteins. This has served as an important analytical tool in AGE research.

Due to the clinical significance of AGE formation, many approaches are being used to diagnose, prevent, or revert AGE formation in the body. The formation of AGE could be inhibited by reacting with an early glycosylation product that results from the original reaction between the target protein and glucose. The inhibition was believed to take place as the reaction between the iahibitor and the early glycosylation product appeared to interrupt the subsequent reaction of the glycosylated protein with additional protein material to form the cross linked late stage product. Compounds like aminoguanidine act to inhibit AGE formation by such mechanism.

The formation of AGE on long-lived proteins is also associated with cross-linking of these proteins. The AGE derived protein crosslinks have been shown to be cleaved by compounds like N- phenacyl thiazolium bromide (PTB), which reacts with and cleaves covalent, AGE derived protein cross links (Vasan et al. Nature 1996; 382: 275-278 ; US 5,853,703, Date of Patent : Dec. 29, 1998). The mechanism of reducing the AGE content in tissues is expected to take place relatively rapidly, in contrast to aminoguanidine, which acts slowly by its very nature of mechanism of action. This current specification is related to compounds of pyridinium class, which break pre-formed AGE, like PTB, and in some cases even more effectively than PTB.

The pyridinium derivative is defined in claim 1, the process for the preparation of pyridinium salt derivative is defined in claim 2, the use of a compound is defined in claim 3, 10 and 11, the pharmaceutical composition is defined in claim 4, the method for the preparation of a parenteral formulation is defined in claim 8.

The main objective of the present invention is to provide a new class of compounds of the pyridinium series which are useful for the management of diabetes and aging related vascular complications and particularly in the treatment of complications of diabetes mellitus and other aging related conditions including kidney disease, nerve damage, atherosclerosis, retinopathy and dermatological conditions. The invention also extends the method to reverse the discoloration of teeth resulting from nonenzymatic browning in the oral cavity which comprises administration of an amount effective to reverse the preformed advanced glycosylation crosslinks, etc.

Another object of the present invention is to provide compounds of the pyridinium series, which exhibit AGE breaking activities.

Yet another object of the present invention is to provide a method of preparation of compounds of the pyridinium series which exhibit AGE breaking activities.

Still another object of the invention is to provide pharmaceutical compositions with a new class of compounds of the pyridinium series according to the invention and their pharmaceutically acceptable salts in combination with suitable carriers, solvents, excepients, diluents and other media normally employed in preparing such compositions.

Still another object of the invention is to provide a method of treatment of a diabetic patient by administration of the compounds of the invention, either singly or in combination with drugs for antidiabetic therapy, or pharmaceutically acceptable salts thereof in required dosage in admixture with pharmaceutically acceptable diluent, solvent, excepients, carriers or other media as may be appropriate for the purpose.

The present invention provides for specific compounds belonging to the class of AGE breakers, of general formula I,

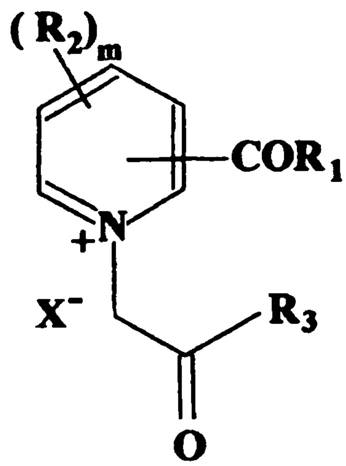

(I)

wherein

$R_1$ is $-R_4-R_5$ or $-N(R_7)\ N\ (R_7)\ R_9$;

$R_4$ is selected from the group consisting of $-N(R_7)R_6O-$,

$-N(R_7)R_6N(R_7)-$, $OR_6O$,

and $-OR_6N(R_7)-$,

where $R_6$ is alkyl;

$R_5$ is selected from the group consisting of alkyl, aryl including heteroaryl,

$-COR_7$, $SO_2R_7$, $-C(S)\ NHR_7$, $-C(NH)NHR_7$, $-COR_{10}$,

$-C(O)NHR_7$ and 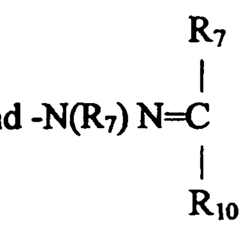

where $R_7$ is selected from the group consisting of H, alkyl and aryl including heteroaryl provided $R_7$ might be different for $R_1$ and $R_3$ in the same compound;

$R_2$ is selected from the group consisting of F, Cl, Br, I, $OR_7$, $NO_2$, alkyl, aryl including heteroaryl, formyl, acyl, $C(O)NR_7R_{10}$, $C(O)OR_7$, $NR_7R_{10}$, $N{=}C(R_7)(R_{10})$, $SR_7$, $SO_2NH_2$, $SO_2$ alkyl and $SO_2$aryl,

and m is 0, 1 or 2;

$R_3$ is selected from the group consisting of $R_7$, $OR_7$, $N(R_7)(R_{10})$, $N{=}C(R_7)(R_{10})$, $N(R_7)N(R_7)(R_{10})$, $N(R_7)\ N{=}C(R_7)(R_{10})$

and $CH(R_7)C(O)R_8$
where $R_8$ is selected from the group consisting of $R_7$, $OR_7$ and $NR_7R_{10}$;
$R_9$ is selected from the group consisting of hydrogen, alkyl, aryl including heteroaryl, $C(O)R_{10}$, $-SO_2R_{10}$, $-C(S)NHR_{10}$, $-C(NH) NH (R_{10})$ and $-C(O) NHR_{10}$,
$R_{10}$ is selected for the group consisting of H, alkyl or aryl including heteroaryl and in each case optionally different from substituent $R_{10}$, provided $R_{10}$ might be different for $R_1$ and $R_3$ in the same compound;
X is selected from group consisting of a halide ion, acetate ion, perchlorate ion, sulfonate ion, oxalate ion, citrate ion, tosylate ion, maleate ion, mesylate ion, carbonate ion, sulfite ion, phosphoric hydrogen ion, phosphonate ion, phosphate ion, $BF_4^-$ and $PF_6^-$ ;

The novel compounds of the invention of general formula I having m as 0 or 1 and - $COR_1$ at position 3 are listed in Table 1A and the novel compounds of the invention of general formula I having m as 0 and - $COR_1$ at position 4 are listed in Table 1B. These compounds can be further defined by their following chemical names :

1-(2-phenylamino-2-oxoethyl)-4- (phenylsulfonyl hydrazino carbonyl) pyridinium chloride (compound 2);

1-(2-ethoxy -2- oxoethyl) -3-(phenylsulfonyl hydrazino carbonyl) pyridinium bromide (compound 3);

1-(2-(2',4'-dichlorophenyl)-2-oxoethyl)-3-(2(methoxy)ethyloxycarbonyl) pyridinium bromide (Compound 4);

1-(2-phenylamino-2-oxoethyl)-3-((benzoyloxy)ethylaminocarbonyl) pyridinium chloride (compound 5);

1-(2-thien-2'-yl- 2-oxoethyl)-3-(phenylaminocarbonyl hydrazinocarbonyl)pyridinium bromide (compound 6);

1-(2-phenyl-2-oxoethyl)-3-(2-(acetoxy)ethylaminocarbonyl) pyridinium bromide (compound 7);

1-(2-phenylamino-2-oxoethyl)-3-(phenyl sulfonyl hydrazino carbonyl) pyridinimn chloride (compound 8);

1-(2-phenylamino-2-oxoethyl)-3-((4-methylphenyl) sulfonyl hydrazino carbonyl)pyridinium chloride (compound 9);

1-(2-phenyl-2-oxoethyl)-3-(2-(benzoyloxy)ethyloxy carbonyl) pyridinium bromide (compound 10);

1-(2-thien-2'-yl-2-oxoethyl)-3-(phenylcarbonyl hydrazino carbonyl) pyridinium bromide (compound 11);

1-(2-ethoxy-2-oxoethyl)-3-((phenylmethyl)sulfonyl hydrazino carbonyl)pyridinium bromide (compound 12);

1-(2-phenyl-2-oxoethyl)-3-((phenylmethyl)sulfonyl hydrazino carbonyl) pyridinium bromide (compound 13);

N, N - bis [3-carbonyl-1-(2-furan-2'-yl-2-oxoethyl) pyridinium] hydrazine dibromide. (Compound No: 14);

N,N'-bis [3-carbonyl-1-(2-thien-2'-yl-2-oxoethyl) pyridinium] hydrazine dichloride.(Compound No: 15);

1-(2-phenylamino-2-oxo ethyl)-3-({2-(1-oxo-3-cyclohexyl)-propyl}-hydrazino-carbonyl]-pyridinium bromide.(Compound No: 17);

1-(2-thien-2'-yl-2-oxoethyl)-3-[2-(benzoyloxy)ethylamino carbonyl]-pyridinium bromide (Compound No: 18);

1-(4-ethoxy-2,4-dioxobutyl)-3-(2-(benzoyloxy)ethylamino carbonyl)-pytidinium chloride. (Compound No: 19);

1-(2',4'-dichloro-phenyl-2-oxoethyl)-3-(2-methoxyethyl aminocarbonyl)-pyridinium bromide. (Compound No: 20);

N,N'-bis-[3-carbonyl-1-(2-cyclopropylamino-2-oxoethyl) pyridinium] hydrazine dichloride. (Compound No: 21);

1-(2-cyclopropylamino-2-oxoethyl)-3-(2-methoxyethylaminocarbonyl)-pyridinium chloride. (Compound No: 22);

1-(2-thien-2'yl-2-oxoethyl)-3-(2-(2-chloro-3-pyridoylhydrazinocarbonyl) -pyridinium chloride. (Compound No: 24);

1-(2-isopropylamino-2-oxoethyl)-3-(2-methylsulfonylhydrazinocarbonyl) -pyridinium chloride. (Compound No: 25);

1-(2-thien-2'-yl-2-oxoethyl)-3-(methanesulfonyl hydrazino carbonyl) pyridinium chloride. (Compound No: 27);

1-(2-thien-2'-yl-2-oxoethyl)-3-((2-methoxy ethyl) amino carbonyl)-5-bromo pyridinium chloride (Compound No: 29);

1-(2'-thien-2'-yl-2-oxoethyl)-3-[1-oxo-1-(2-methoxy carbonyl)pyridyl] hydrazino pyridinium chloride. (Compound No: 30);

1-[1-(2-thien-2'-yl-2-oxoethyl)-6-methyl-3-carbonyl pyridinium]-2-[1-(2-Thien-2'-yl-2-oxoethyl)-3-carbonyl pyridinium] hydrazine dichloride(compound no: 31).

1-(2-thien-2'-yl-2-oxoethyl)-3-(isopropylsulfonyl hydrazino carbonyl) pyridinium bromide(compound no: 32).

1-(2-(4-benzyl piperidin-1-yl)-2-oxoethyl)-3-(methanesulfonyl hydrazino carbonyl) pyridinium chloride(compound no: 33).

1-(2-(2-ethoxy carbonyl pyrrolidin-1-yl)-2-oxoethyl)-3-(methanesulfonyl hydrazino carbonyl) pyridinium chloride. (compound no: 34).

1-(2-thien-2'-yl-2-oxoethyl)-3-(methanesulfonyl hydrazino carbonyl)-5-bromo pyridinium bromide. (compound no: 35).

1-(2-thien-2'-yl-2-oxoethyl)-3-(ethoxycarbonyl hydrazino carbonyl) pyridinium bromide. (compound no: 36).

1-(2-(5-chloro-thien-2-yl)-2-oxoethyl)-3-(methanesulfonyl hydrazino carbonyl) pyridinium bromide (compound no: 37).

N,N'-bis[3-carbonyl-1-(2-(4-nitro-thien-2-yl)-2-oxoethyl)pyridinium] hydrazine dichloride. (compound no: 38).

1-(2-thien-2'-yl 2-oxoethyl)-3-(methanesulfonyl hydrazino carbonyl) -6-methyl pyridinium bromide. (compound no: 39).

N,N'-bis[3-carbonyl-1-(2-(5-methyl-thien-2-yl)-2-oxoethyl) pyridinium] hydrazine dichloride. (compound no: 40).

N,N'-bis[3-carbonyl-1-(2-(2-ethoxycarbonyl pyrrolidin-1-yl)-2-oxoethyl) pyridinium] hydrazine dichloride. (compound no: 41).

1-[1-(2-thien-2'-yl-2-oxoethyl)-5-aminocarbonyl-3-carbonyl pyridinium]-2-[1-(2-Thien-2'-yl-2-oxoethyl)-3-carbonyl pyridinium] hydrazine dichloride (compound no: 42).

1-(2-(4-carbethoxy-thiazolidin-3-yl)-2oxoethyl)-3-(methanesulfonyl hydrazino carbonyl) pyridinium chloride (compound no: 43).

N,N'-bis[3-carbonyl-1-(2-(5-chloro-thien-2-yl)-2-oxoethyl) pyridinium] hydrazine dichloride. (compound no: 44).

1-(2-(5-methyl-thien-2-yl)-2-oxoethyl)-3-(methanesulfonyl hydrazino carbonyl) pyridinium chloride (compound no: 45).

1-(2-(4-nitro-thien-2-yl)-2-oxoethyl)-3-(methanesulfonyl hydrazino carbonyl) pyridinium bromide. (compound no: 46).

1-(2-phenylamino-2-oxoethyl)-3-(phenyl hydrazino carbonyl) pyridinium chloride (compound no: 47).

1-(2-phenylamino-2-oxoethyl)-4-[2-(benzoyloxy) ethylamino carbonyl] pyridinium chloride (compound no: 48).

1-2-(5-nitro-thien-2-yl)-2-oxoethyl)-3-(methanesulfonyl hydrazino carbonyl) pyridinium chloride (compound no: 49).

1-(2-thien-2'-yl-2-oxoethyl)-3-(trifluoromethanesulfonyl hydrazino carbonyl) - pyridinium bromide. (compound no.

50).

1-(2-thien-2'-yl-2-oxoethyl)-3-(phenyl hydrazino carbonyl) pyridinium bromide (compound no.51).

1-(2-thien-2'-yl-2-oxoethyl)-3-(p-methoxy phenyl sulfonyl hydrazino carbonyl) pyridinium bromide (compound no. 52).

1-(2-ethoxy-2-oxoethyl)-3-(phenyl aminocarbonyl hydrazino carbonyl) pyridinium bromide (compound no. 53).

1-(2-ethoxy-2-oxoethyl)-3-(p-toluene sulfonyl hydrazino carbonyl) pyridinium bromide (compound no. 54).

1-(2-phenyl-2-oxoethyl)-3-(phenylamino carbonyl hydrazino carbonyl) pyridinium bromide (compound no. 55).

1-(2-phenylamino-2-oxoethyl)-3-(benzyl sulfonyl hydrazino carbonyl) pyridiniumchloride. (compound no. 56).

1-(2-phenyl-2-oxoethyl)-4-(methanesulfonyl hydrazino carbonyl) pyridinium bromide (compound no. 57).

1-(2-phenyl-2-oxoethyl)-3-(phenyl hydrazino carbonyl) pyridinium bromide. (compound no. 58).

1-(2-ethoxy-2-oxoethyl)-4-[2-(benzoyloxy) ethyl amino carbonyl] pyridinium bromide (compound no. 59).

1-(2-ethoxy-2-oxoethyl)-3-(phenyl hydrazino carbonyl) pyridinium bromide (compound no. 60).

1-(2-phenyl-2-oxoethyl)-3-(p-methoxyphenyl sulfonyl hydrazino carbonyl) pyridinium bromide. (compound no. 61).

1-(2-phenyl-2-oxoethyl)-4-[2-(benzoyloxy) ethyl amino carbonyl] pyridinium bromide. (compound no. 62).

1-(2-ethoxy-2-oxoethyl)-4-(p-methanesulfonyl hydrazino carbonyl) pyridinium bromide. (compound no. 63).

Table 1A -

| **Pyridinium derivatives (having m as 0 or 1 and $-COR_1$ at position 3)** | | | | |
|---|---|---|---|---|
| Compound No. | $R_1$ | $-R_2$ | $-R_3$ | -X |
| 3 | $-NHNH-SO_2Ph$ | - | OEt | Br |
| 4 | $-OCH_2CH_2OCH_3$ | - | 2,4-dichlorophenyl | Br |
| 5 | $-NHCH_2CH_2OCOPh$ | - | NH phenyl | Cl |
| 6 | -NHNHCONHPh | - | 2-thienyl | Br |
| 7 | $NHCH_2CH_2OCOCH_3$ | - | phenyl | Br |
| 8 | $NHNHSO_2Ph$ | - | NH phenyl | Cl |
| 9 | $NHNHSO_2Ph(4-CH_3)$ | - | NH phenyl | Cl |
| 10 | $OCH_2CH_2OCOPh$ | - | phenyl | Br |
| 11 | -NHNHCOPh | - | 2-thienyl | Br |
| 12 | $NHNHSO_2CH_2Ph$ | - | OEt | Br |
| 13 | $NHNHSO_2CH_2Ph$ | - | phenyl | Br |
| 14 | Structure (a) | - | 2-furyl | Br |
| 15 | Structure-(b) | - | 2-thienyl | Cl |
| 17 | $NHNHCOCH_2CH_2$-cyclohexyl | - | NH-phenyl | Cl |
| 18 | $NHCH_2CH_2OCO$-phenyl | - | 2-thienyl | Br |

Table 1A - (continued)

| **Pyridinium derivatives (having m as 0 or 1 and $-COR_1$ at position 3)** | | | | |
|---|---|---|---|---|
| Compound No. | $R_1$ | $-R_2$ | $-R_3$ | -X |
| 19 | $NHCH_2CH_2OCO$-phenyl | - | $CH_2CO_2$-ethyl | Cl |
| 20 | $-NHCH_2CH_2OCH_3$ | - | -2,4-dichlorophenyl | Br |
| 21 | Structure-(c) | - | NH-cyclopropyl | Cl |
| 22 | $-NHCH_2CH_2OCH_3$ | - | NH-cyclopropyl | Cl |
| 24 | Structure-(e) | - | 2-thienyl | Cl |
| 25 | $NHNHSO_2CH_3$ | - | NH-isopropyl | Cl |
| 27 | $NHNHSO_2CH_3$ | - | 2-thienyl | Cl |
| 29 | $NHCH_2CH_2OCH_3$ | - | 2-thienyl | Cl |
| 30 | Structure-(g) | - | 2-thienyl | Cl |
| 31 | Structure - (h) | - | 2-thienyl | Cl |
| 32 . | $-NHNHSO_2$isopropyl | - | 2-thienyl | Br |
| 33 | $-NHNHSO_2CH_3$ | - | Structure (i) | Cl |
| 34 | $-NHNHSO_2CH_3$ | - | Structure (j) | Cl |
| 35 | $-NHNHSO_2CH_3$ | 5-Bro mo | 2-thienyl | Br |
| 36 | $-NHNHCOC_2H_5$ | - | 2-thienyl | Br |
| 37 | $-NHNHSO_2CH_3$ | - | 5-chloro-2-thienyl | Br |
| 38 | Structure (k) | - | 4-nitro-2-thienyl | Cl |
| 39 | $-NHNHSO_2CH_3$ | 6-met hyl | 2-thienyl | Br |
| 40 | Structure (l) | - | 5-methyl-2-thienyl | Cl |
| 41 | Structure (m) | - | Structure (j) | Cl |
| 42 | Structure (n) | - | 2-thienyl | Cl |
| 43 | $-NHNHSO_2CH_3$ | - | Structure (o) | Cl |
| 44 | Structure (p) | - | 5-chloro-2-thienyl | Cl |
| 45 | $-NHNHSO_2CH_3$ | - | 5-methyl-2-thienyl | Cl |
| 46 | $- NHNHSO_2CH_3$ | - | 4-nitro-2-thienyl | Br |
| 47 | -NHNHPh | - | -NHPh | Cl |
| 49 | $-NHNHSO_2CH_3$ | - | 5-nitro-2-thienyl | Cl |
| 50 | $-NHNHSO_2CF_3$ | - | 2-thienyl | Br |
| 51 | -NHNHPh | - | 2-thienyl | Br |
| 52 | $-NHNHSO_2$-4-methoxy-Phenyl | - | 2-thienyl | Br |
| 53 | -NHNHCONHPh | - | -OEt | Br |
| 54 | $-NHNHSO_2$-4-methyl-Phenyl | - | -OEt | Br |
| 55 | -NHNHCONHPh | - | Ph | Br |
| 56 | $-NHNHSO_2CH_2Ph$ | - | -NHPh | Cl |
| 58 | -NHNHPh | - | Ph | Br |

Table 1A - (continued)

| Pyridinium derivatives (having m as 0 or 1 and $-COR_1$ at position 3) | | | | |
|---|---|---|---|---|
| Compound No. | $R_1$ | $-R_2$ | $-R_3$ | -X |
| 60 | -NHNHPh | - | -OEt | Br |
| 61 | $-NHNHSO_2$-4-methoxy-Phenyl | - | Ph | Br |

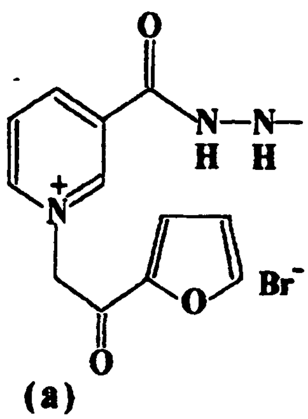

(a)

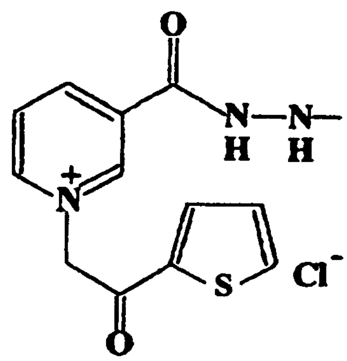

(b)

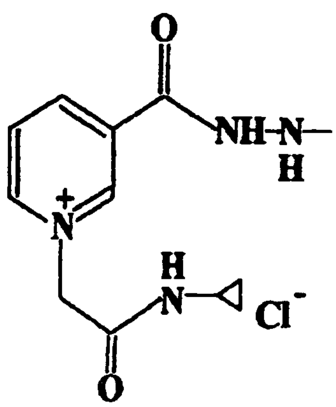

(c)

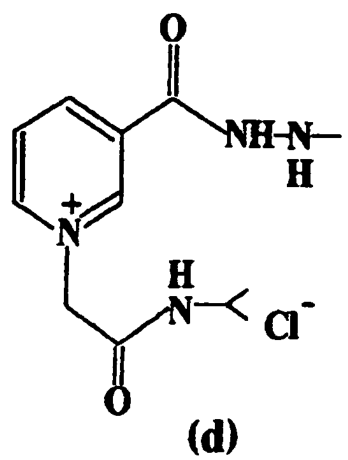

(d)

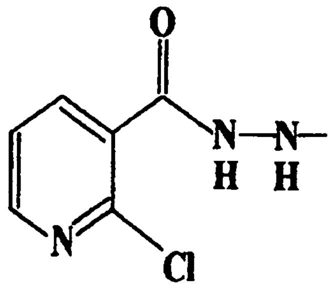

(e)

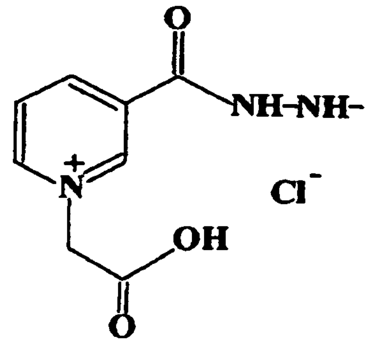

(f)

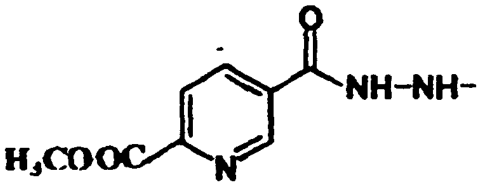

Structure (g)

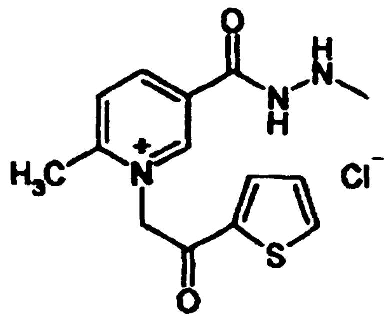

Structure (h)

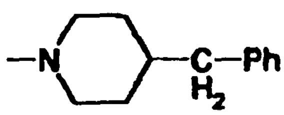

Structure (i)

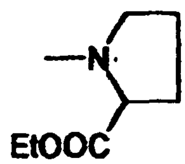

Structure (j)

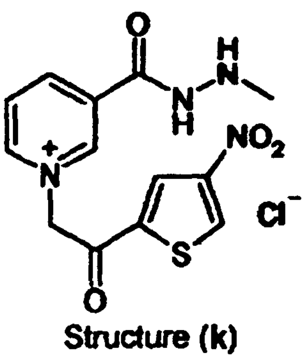

Structure (k)

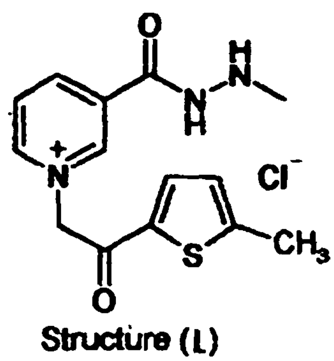

Structure (L)

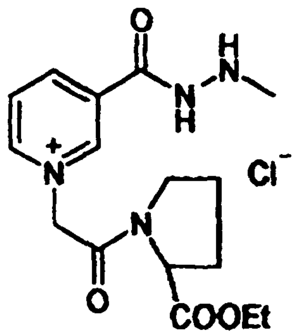

Structure (m)

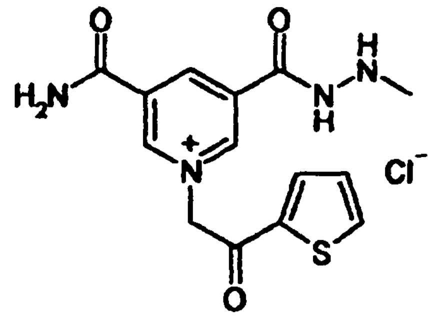

Structure (n)

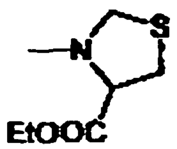

Structure (o)

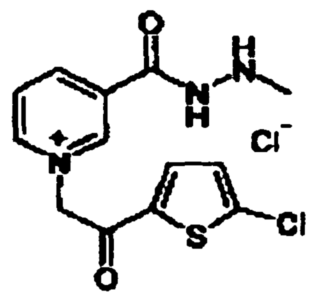

Structure (P)

Table 1B -

| Pyridinium derivatives (having m as 0 and $-COR_1$ at position 4) | | | | |
|---|---|---|---|---|
| Compound | $-R_1$ | $-R_2$ | $-R_3$ | -X |
| 2 | $NHNHSO_2Ph$ | - | NH phenyl | Cl |
| 48 | $-NHCH_2CH_2OCOPh$ | - | -NHPh | Cl |
| 57 | $-NHNHSO_2CH_3$ | - | -Ph | Br |
| 59 | $-NHCH_2CH_2OCOPh$ | - | -OEt | Br |
| 62 | $-NHCH_2CH_2OCOPh$ | - | -Ph | Br |
| 63 | $-NHNHSO_2CH_3$ | - | -OEt | Br |

According to the embodiment of the present invention, the present compounds are used for the treatment of diabetic complications, and aging related complications including kidney disease, nerve damage, atherosclerosis, retinopathy, dermatological conditions and colouration of teeth occurring due to the higher levels of preformed AGE. The increased levels of preformed AGE can be brought under control by breaking the AGE products using compounds mentioned in the invention.

The invention also provides a process for the preparation of novel compounds of the pyridinium series.

The said process for the preparation of compound 15, comprises, adding a solution of 2-chloro acetylthiophene in isopropanol to N,N-bis-(nicotinyl)hydrazine dissolved in methanol, refluxing for six hours, cooling, filtering the precipitated solid, washing the solid with hot ethyl acetate and finally purifying the solid with 20 ml of methanol : ethyl acetate (3 : 1) to yield the desired compound.

Similarly, the other novel compounds are prepared from properly substituted pyridine derivatives followed by quarternization with appropriate reagent by refluxing in alcoholic solvents like, methanol, ethanol, propanol, etc and high boiling solvents like toluene or xylene etc, for 6 - 48 hrs. to give the desired compounds.

The examples of substituted pyridine derivatives which can be used for preparation of specific compounds of the invention are given below:

1. N,N'-bis(nicotinyl)hydrazine
2. 3-[(2-pyridyl)hydrazinocarbonyl]pyridine
3. 3-[(2-methanesulfonyl)hydrazinocarbonyl]pyridine
4. 3-[(2-benzoyloxy)ethylaminocarbonyl]pyridine
5. 3-[(2-phyenylsulfonyl)hydrazinocarbonyl]pyridine
6. 3-[(2-acetoxy)ethyloxycarbonyl]pyridine
7. 3-[(2-benzoyloxy)ethyloxycarbonyl]pyridine
8. 3-[(2-methoxy)ethyloxycarbonyl]pyridine
9. 3-[(2-phyenylaminocarbonyl)hydrazinocarbonyl]pyridine
10. 3-[(2-acetoxy)ethylaminocarbonyl]pyridine
11. 3-[(2-(4-methylphenyl sulfonyl)hydrazinocarbonyl)]pyridine
12. 3-[(2-benzoyl)- hydrazino carbonyl]pyridine
13. 3-[(2-phenylmethane sulfonyl) hydrazino carbonyl]pyridine
14. 3-[(2-(3- cyclohexylpropanoyl) hydrazino carbonyl]pyridine
15. 3-[(2-methoxy)ethylaminocarbonyl]pyridine
16. 3-[1-oxo-1-(2-methoxycarbonyl)pyridyl]hydrazino pyridine

The examples of quaternizing agents which may be used in the reaction are given below:

1. 2-bromoacetyl thiophene
2. 2-chloroacetyl thiopene
3. phenacylbromide
4. phenacylchloride
5. 2,4-dichloropheanacylbromide
6. N- phenyl chloroacetamide
7. N- cyclopropyl chloroacetamide
8. ethylbromoacetate
9. bromo acetylfuran
10. N- isopropylchloroacetamide
11. N- chloroacetyl-2-pyrrolidinone
12. chloroacetic acid

**In-vitro screening for AGE-breaking Activity**

The in vitro AGE formation, studied in the laboratory, by incubating reducing sugar glucose, with protein bovine serum albumin, resulted in browning of solution and increase in the fluorescence. Fluorescence was used as the criteria to monitor the increased AGE formation.

**Example 1**

**AGE breaker activity has been confirmed by the screening procedure as mentioned below:**

Materials:

Bovine serum albumin (fraction V) (BSA)
Glucose, analytical grade
Phosphate buffered saline (PBS)

Equipment:

Microplate ELISA Reader - Spectramax Plus (Molecular Devices, USA)
Microplate washer, (Bio -Tec Instruments, USA)
pH meter

Methods of experiment: Elisa (Enzyme Linked Immunosorbent Assay) 160 mg/ml of protein, bovine serum albumin, BSA and 1.6M glucose sugar were dissolved in phosphate buffered saline, PBS. Sodium azide was added at 0.02% concentration as a preservative. The solution was filtered asceptically through a 0.22 μM filter and kept for aging at 37°C for 16 weeks. After 16 weeks the solution was dialyzed against PBS, aliquoted and stored at - 20°C.

To determine the AGE breaking activity, 10μg/ml of the 16 weeks AGE-BSA was incubated with different concentrations of the test compounds at 37°C for 24 hours and AGE breaking activity of the test compounds by ELISA was determined.

ELISA was performed as follows:

1. Different concentrations of 16 weeks AGE-BSA were coated on a microtitre plate as standard. Each concentration is coated in triplicates.
2. The test samples were coated on microtitre plate at a concentration of 5 ng. to 20 ng per well in triplicates.
3. The plate was incubated at 37°C for one hour.
4. After incubation the plate was washed with PBST (PBS with 0.05% Tween 20).
5. Blocking with 5% skimmed milk in PBS at 37°C for one hour was done.
6. The plate was washed with PBST.
7. Primary antibody against AGE-BSA was added and the plate is incubated at 37°C for one hour.
8. The plate was washed with PBST
9. Secondary antibody anti rabbit HRPO (Horse-Radish Per Oxidase) conjugate was added and the plate is incubated at 37°C for one hour.
10. The plate was washed with PBST.
11. Colour development with OPD (orthophenylenediamine dihydrochloride) and hydrogen peroxide was done.
12. OD (optical density) at (450nm reading - 620nm reading) was measured after incubation at 37°C for 15 minutes with Microplate ELISA Reader.

The breaker activity of the compounds were determined by the following formula:

$$\%\ \text{Breaker activity} = \frac{OD_{450\text{-}620}\text{Control} - OD_{450\text{-}620}\text{Test}}{OD_{450\text{-}620}\text{Control}} \times 100$$

$OD_{450\text{-}620}$Control= Absorbance of 20ng AGE-BSA after incubation at 37°C for 24 hours without test compound
$OD_{450\text{-}620}$Test= Absorbance of 20ng AGE-BSA after incubation at 37°C for 24 hours with required concentration of test compound

Using specific examples, the % AGE breaking activity was calculated and recorded in Table 2.

Table 2

| Sample | Concentration (mM) | % Breakage |
|---|---|---|
| PTB | 10 | 27 |
| | 20 | 47 |
| Compound 4 | 10 | 13 |
| | 25 | 41 |
| Compound 5 | 10 | 37 |
| | 25 | 90 |
| Compound 14 | 10 | 14 |
| Compound 15 | 5 | 20 |
| Compound 20 | 5 | 17.66 |
| Compound 21 | 5 | 22.8 |
| Compound 22 | 10 | 12.38 |
| Compound 24 | 10 | 12.51 |

Table 2 (continued)

| Sample | Concentration (mM) | % Breakage |
|---|---|---|
| Compound 25 | 10 | 10.85 |
| Compound 27 | 10 | 17.53 |
| Compound 29 | 10 | 32.38 |
| Compound 31 | 2.5 | 85.67 |
| Compound 32 | 10 | 31.45 |
| Compound 33 | 10 | 20.94 |
| Compound 34 | 10 | 25.34 |
| Compound 35 | 2.5 | 29.36 |
| Compound 36 | 10 | 33.43 |
| Compound 37 | 10 | 40.85 |
| Compound 38 | 10 | 75.92 |
| Compound 39 | 1.0 | 77.69 |
| Compound 40 | 10 | 81.95 |
| Compound 41 | 10 | 20.31 |
| Compound 42 | 1 | 95.36 |
| Compound 43 | 10 | 25.06 |
| Compound 44 | 10 | 78.41 |
| Compound 45 | 10 | 25.17 |
| Compound 46 | 10 | 60.94 |
| Compound 47 | 2.5 | 68.35 |
| Compound 48 | 10 | 19.07 |
| Compound 49 | 1 | 42.01 |
| Compound 50 | 10 | 92.64 |

Hence compounds 5, 29,31,32, 35, 36-40, 42, 44, 46,47, 49 and 50 have superior AGE breaking activity compared to PTB, of which the potency of compounds 31, 38-40, 42, 44, 46, 47, 49 and 50 are significantly much higher.

**In-vivo screening for AGE-breaking Activity :**

The test compounds were studied for their beneficial effects on diabetic neuropathy and nephropathy in a rat model of diabetes. The rats were divided into three groups. The first group consisted of age matched untreated non-diabetic animals. The second group consisted of diabetic controls and the third group was the diabetic group treated with the test compound. Each treatment group had its own corresponding control and diabetic groups. The second and third groups were treated with Streptozotocin (STZ) at 60mg/kg for the induction of diabetes. After completion of 12 weeks of diabetes the rats were treated with the test compound daily (doses shown in table) for a period of 8 weeks. At the end of the treatment the creatinine clearances and nerve conduction velocities (NCV) of the animals were estimated.
Creatinine clearances of the rats were estimated as follows

$$\text{Creatinine clearance} = \frac{\text{Concentration of creatinine in the urine}}{\text{Concentration of creatinine in the blood.}} \times \text{ml urine passed/minute}$$

The creatinine clearance in untreated diabetic group was compared with the treated group and the percentage improvements are shown in the Table 3.

The nerve conduction velocity was measured using a modified method of Biro et al 1998. Briefly under ether anesthesia the sciatic and tibial nerves were electrically stimulated at the sciatic notch or ankle, respectively. Electromyograms (EMG's) recorded from the plantar muscles consisted of two components: (1) the short latency direct motor response (M) and the monosynaptically elicited long-latency sensory response (H, Hoffmann reflex). Latency and the duration of the M responses were measured and the motor nerve conduction velocity (MNCV) was calculated as follows :

$$\text{MNCV} = \frac{\text{Distance between the sciatic and tibial stimulation points}}{\text{Differences of the latency for } M_{\text{sciatic}} \text{ and } M_{\text{tibial}}}.$$

The percentage improvement in the nerve conduction velocities in the group treated with the test compounds was calculated as follows:

$$\%\ \text{Improvement in the NCV's}$$

$$= \frac{\text{NCV of the treated group - NCV of the diabetic group}}{\text{NCV of the control group - NCV of the diabetic group.}}$$

Table 3

| **Effect of compound Nos 14 and 21 on the creatinine clearance and nerve conduction velocities:** | | |
|---|---|---|
| **Parameters** | **Compound No.15 (7.5 mg/kg, b.i.d.)** | **Compound No.21 (6.0 mg/kg, b.i.d.)** |
| **% Increase in creatinine clearance** | **103.0** | **5.0** |
| **% Increase in the NCV** | **60.0** | **58.4** |

The results show that compounds of this class have beneficial effects on creatinine clearance and nerve conduction velocities.

**Discussion of the test results:**

All the test compounds mentioned in the current application have shown an *invitro* AGE-breaker effect. Under conditions of chronic hyperglycemia in rats there is a spontaneous non-enzymatic reaction between glucose, lipids and proteins that leads to the formation of advanced glycosylation end products. In this animal model decreased creatinine clearance and decreased nerve conduction velocity have been demonstrated. These changes are related to damage to renal and neuronal tissues. During chronic NIDDM patients, there is a decrease in the creatinine clearances as a manifestation of the diabetes induced renal damage. One of the major factors contributing to renal damage is the glycation of the long-lived proteins in the kidney. It is well recognized that there is a decrease in the nerve conduction velocities in chronic diabetic subjects, which is a manifestation of neuropathy. Breaking of cross-linked proteins in the neuronal tissues and associated vasculature could lead to an improvement in the neuronal function.

The compounds of the present invention have shown a functional improvement both in terms of the improvement in the creatinine clearance and an improvement in the nerve conduction velocities. The evidences stated above clearly demonstrate that these compounds could play a major role in the prevention and treatment of various diabetic and aging related complications like nephropathy and neuropathy.

The following examples give method of preparation of the specific novel compounds of the invention as given in Table 1.

**Reference Example**

**1-(2-Ethoxy-2-oxoethyl)-4-(phenylsulfonyl hydrazino carbonyl) pyridinium bromide:**

To a boiling solution of N-(benzene sulfonyl)-isonicotinic hydrazide (1.0g.,0.036 mol.) in isopropanol (25 ml),

a solution of ethyl bromo acetate (0.6g., 0.036 mol.) in 10ml isopropanol was added and the reaction mixture was refluxed for 24 hrs. The reaction mixture was concentrated under vacuum (~10ml.) and was recrystalized from a mixture of methanol-ethyl acetate (3:1, 15ml.) to afford the solid. The solid was further washed with (10ml.) ethyl acetate yielding 1.05 g of required product.

| | |
|---|---|
| Yield | 60% |
| m.p. | 171 - 173°C. |
| IR (KBr, $cm^{-1}$) | 1745, 1685, 1645. |
| $^1$HNMR (DMSO$d_6$, 400 MHz) δ | 11.41 (1H, s), 10.39 (1H, s), 9.10 (2H, d), 8.27 (2H, d), 7.82 - 7.80 (2H, d), 7.60 - 7.57 (1H, t), 7.50 - 7.46 (2H, t), 5.63 (2H, s), 4.18 - 4.12 (2H, q), 1.19 - 1.15 (3H, t). |
| Mass (m/z) | 364, 365, 366 |

According to the above mentioned procedure, the following compounds are synthesized by reacting the corresponding pyridine derivatives with appropriate reagents by refluxing in methanol, ethanol, propanol, toluene of xylene for 6 - 48 hrs. to get the desired compounds.

**Example 3**

**1-(2-Phenylamino-2-oxoethyl)-4-(phenylsulfonyl hydrazino carbonyl) pyridinium chloride (Compound 2):**

| | |
|---|---|
| Yield | 10% |
| m.p. | 225 - 227°C. |
| IR (KBr, $cm^{-1}$) | 1693, 1642, 1592 |
| $^1$HNMR(DMSO$d_6$, 400 MHz) δ | 11.55 (1H, s), 10.99 (1H, s), 10.49 (1H, s), 9.20 (2H, d), 8.34 (2H, d), 7.89 (2H, d), 7.73 - 7.64 (1H, t), 7.61 - 7.56 (4H, m), 7.37 - 7.33 (2H, t), 7.12 - 7.09 (1H, t), 5.73 (2H, s). |
| Mass (m/z) | 411, 412, 413, 414 |

**Example 4**

**1-(2-Ethoxy-2-oxoethyl)-3-(phenylsulfonylhydrazino carbonyl) pyridinium bromide (Compound 3):**

| | |
|---|---|
| Yield | 75% |
| m.p. | 145 - 147 °C. |
| IR(KBr $cm^{-1}$) | 1744, 1713, 1633 |
| $^1$HNMR (DMSO$d_6$, 400MHz) δ | 11.27(1H,s),10.36 (1H, s), 9.28(1H,s), 9.09(1H,d), 8.83(1H,d),8.27 - 8.24 (1H,m), 7.82 - 7.79 (2H,m), 7.58 (1H,t), 7.48 (2H, t), 5.59 (2H, s), 4.17 - 4.12 (2H, q), 1.16 (3H,t). |
| Mass (m/z) | 364, 365, 366 |

**Example 5**

**1-(2-(2',4'-Dichlorophenyl)-2-oxoethyl)-3-(2(methoxy)ethyloxycarbonyl) pyridinium bromide (Compound 4):**

| | |
|---|---|
| Yield | 25% |
| m.p. | 156 - 158°C. |
| IR (KBr, $cm^{-1}$) | 1731, 1706, 1640 |

(continued)

| | |
|---|---|
| $^1$HNMR (DMSO $d_6$,400 MHz)δ | 9.61 (1H, s),9.20(1H, d),9.13 (1H,d), 8.45 - 8.41 (1H, m),8.15 (1H, d),7.92(1H, d),7.78 - 7.76 (1H, m), 6.49 (2H, s), 4.56 - 4.54 (2H, m), 3.72 - 3.69 (2H, q), 3.31 (3H, s). |
| Mass (m/z) | 368, 369, 370, 371 |

**Example 6**

**1-(2-Phenylamino-2-oxoethyl)-3-(2-(benzoyloxyl) ethylaminocarbonyl) pyridinium chloride (Compound 5):**

| | |
|---|---|
| Yield | 70% |
| m.p. | 171 -172°C |
| IR (KBr, $cm^{-1}$) | 1720, 1692, 1668 |
| $^1$HNMR : (DMSO$d_6$, 400 MHz) δ | 11.06 (1H, s), 9.67 (1H, t), 9.59 (1H, s), 9.20 (1H,d), 9.11 (1H, d), 8.36 - 8.32 (1H, m), 8.00 (2H, d), 7.66 - 7.61 (3H, m),7.51 (2H, t),7.34 (2H, t), 7.10 (1H, t), 5.77 (2H,s), 4.45 (2H,t), 3.76 - 3.72 (2H, q). |
| Mass (m/z) | 404, 405, 406, 407 |

**Example 7**

**1-(2-Thien-2'-yl-2-oxoethyl)-3-(phenylaminocarbonyl hydrazinocarbonyl) pyridinium bromide (Compound 6):**

| | |
|---|---|
| Yield | 30% |
| m.p. | 202 - 204°C. |
| IR (KBr, $cm^{-1}$) | 1718, 1673 |
| $^1$HNMR : (DMSO$d_6$, 400 MHz) δ | 11.03 (1H, s), 9.55 (1H, s), 9.18 (1H, d), 9.10 (1H, d), 9.00 (1H, s),8.57 (1H,s), 8.46 - 8.42 (1H, t), 8.25 - 8.22 (2H, m), 7.47 - 7.45 (2H, d), 7.43 - 7.41 (1H, t), 7.29 -7.25 (2H, t), 7.0 - 6.96 (1H, t), 6.46 (2H, s). |
| Mass (m/z) | 381,382,383 |

**Example 8**

**1-(2-Phenyl-2-oxoethyl)-3-(2-(acetoxy) ethylaminocarbonyl) pyridinium bromide (Compound 7):**

| | |
|---|---|
| Yield | 55% |
| m.p. | 186 - 188 |
| IR (KBr, $cm^{-1}$) | 1734, 1697, 1679 |
| $^1$HNMR (DMSO$d_6$, 400 MHz) δ | 9.47(1H,s), 9.36 (1H,t), 9.13 - 9.05 (2H, m), 8.42 - 8.38 (1H, m), 8.06 (2H, d), 7.80 (1H, t), 7.67 (2H, t), 6.54 (2H, s), 4.18 (2H,t), 3.61 - 3.57 (2H,q), 2.02 (3H,s). |
| Mass (m/z) | 327, 328, 329. |

**Example 9**

**1-(2-Phenylamino-2-oxoethyl)-3-(phenyl sulfonyl hydrazino carbonyl) pyridinium chloride (Compound 8):**

| | |
|---|---|
| Yield | 38% |

(continued)

| | |
|---|---|
| m.p. | 232 - 234°C. |
| IR (KBr, $cm^{-1}$) | 1689, 1636, 1596 |
| $^1$HNMR (DMSO$d_6$, 400 MHz) δ | 11.30 (1H, s), 10.80 (1H, s), 10.37 (1H, s), 9.29 (1H, s), 9.09 (1H, d), 8.81 (1H, d), 8.25 - 8.21 (1H, t), 7.82 - 7.80 (2H, d), 7.59 - 7.46 (5H, m), 7.28 - 7.24 (2H, t), 7.04 -7.00 (1H, t), 5.62 (2H,s). |
| Mass (m/z) | 411, 412, 413, 414 |

**Example 10**

**1-(2-Phenylamino-2-oxoethyl)-3-((4-methylphenyl)sulfonyl hydrazino carbonyl) pyridinium chloride (Compound 9):**

| | |
|---|---|
| Yield | 48% |
| m.p. | 205 - 206°C |
| IR(KBr, $cm^{-1}$) | 1712, 1681, 1632 |
| $^1$HNMR (DMSO$d_6$, 400 MHz) δ | 11.35 (1H, s), 10.86 (1H, s), 10.36 (1H, s), 9.38 (1H, s), 9.17 (1H, d), 8.90 (1H, d), 8.34 - 8.30 (1H, m), 7.78 - (2H,d), 7.59 (2H, d), 7.37 - 7.33 (4H,m), 7.11 (1H, t), 5.70 (2H,s), 2.36 (3H, s). |
| Mass (m/z) | 425, 426, 427, 428 |

**Example 11**

**1-(2-Phenyl-2-oxoethyl)-3-(2-(benzoyloxy)ethyloxy carbonyl) pyridinium bromide (Compound 10):**

| | |
|---|---|
| Yield | 35% |
| m.p. | 132 -134°C. |
| IR (KBr, $cm^{-1}$) | 1730, 1705, 1690 |
| $^1$HNMR (DMSO$d_6$, 400 MHz) δ | 9.80 (1H, s), 9.36 (1H, d), 9.30 (1H, d), 8.58 (1H, t), 8.21 (2H, d), 8.12 (2H, d), 7.95 (1H, t), 7.85 - 7.80 (3 H, m), 7.68 (2H, t), 6.71 (2H, s), 4.95 - 4.93 (2H, m), 4.82 - 4.80 (2H, m). |
| Mass (m/z) | 390, 391, 392. |

**Example 12**

**1-(2-Thien-2'-yl-2-oxoethyl)-3-(phenylcarbonyl hydrazino carbonyl) pyridinium bromide (Compound 11):**

| | |
|---|---|
| Yield | 45% |
| m.p. | 80 - 81 °C |
| IR(KBr $Cm^{-1}$) | 1700, 1663, 1631 |
| $^1$HNMR (DMSO$d_6$, 400MHz) δ | 11.49 (1H, s), 10.95 (1H, s), 9.67 (1H, s), 9.34 (1H, d), 9.27 (1H, d), 8.52 - 8.48 (1H, m), 8.29 - 8.28 (2H, m), 8.00 (2H, d), 7.68 (1H, t), 7.59 (2H, t), 7.46 (1H, t), 6.63 (2H,s) |
| Mass (m/z) | 366, 367, 368, 369 |

**Example 13**

**1-(2-Ethoxy-2-oxoethyl)-3-((phenylmethyl)sulfonyl hydrazino carbonyl) pyridinium bromide (Compound 12):**

| | |
|---|---|
| Yield | 50% |
| m.p. | 147 - 148°C |
| IR (KBr, $cm^{-1}$) | 1749, 1698, 1640 |
| $^1$HNMR (DMSO$d_6$, 400 MHz) δ | 11.57 (1H, s), 10.21 (1H,s), 9.75 (1H,s), 9.38 (1H, d), 9.24 (1H, d), 8.59 - 8.56 (1H, m), 7.67 - 7.65 (2H, m), 7.58 - 7.52 (3H, m), 5.90(2H, s),4.68 (2H, s), 4.45 - 4.39(2H, q),1.43 (3H, t). |
| Mass (m/z) | 377, 378, 379 |

**Example 14**

**1-(2-Phenyl-2-oxoethyl)-3-((phenylmethyl)sulfonyl hydrazino carbonyl)pyridinium bromide (Compound 13):**

| | |
|---|---|
| Yield | 80% |
| m.p. | 205 - 207° C |
| IR (KBr, $Cm^{-1}$) | 1657, 1637 |
| $^1$HNMR (DMSO$d_6$, 400 MHz) δ | 11.59 (1H,s), 10.20 (1H,s), 9.71 (1H,s), 9.33 (1H, d), 9.27 (1H, d), 8.62 - 8.59 (1H, m), 8.25 - 8.23 (2H, d), 7.99 -7.95 (1H, t), 7.86 - 7.82 (2H, t), 7.67 - 7.65 (2H, m), 7.57 - 7.52 (3H, m),6.72 (2H, s), 4.69 (2H, s). |
| Mass (m/z) | 410, 411, 412, 413 |

**Example 15**

**N, N' - Bis [3-carbonyl-1-(2-furan-2'-yl-2-oxoethyl) pyridinium] hydrazine dibromide. (Compound No: 14)**

| | |
|---|---|
| Yield | 23% |
| m.p. | 267-269 °C (dec) |
| IR (KBr, $cm^{-1}$) | 1687,1660 |
| $^1$H NMR (DMSO $d_6$, 400 MHz) ä | 11.65 (2H,s), 9.56 (2H,s), 9.21 - 9.15 (4H,m), 8.48-8.44 (2H,t), 8.23 (2H,s), 7.74 - 7.73 (2H,d), 6.91 - 6.90 (2H,d) 6.34 (4H,s) |
| Mass (m/z) | 459, 460, 461 |

**Example 16**

**N,N'-Bis [3-carbonyl-1-(2-thien-2'-yl-2-oxoethyl) pyridinium] hydrazine dichloride.(Compound No: 15)**

| | |
|---|---|
| Yield | 35% |
| m.p. | 275-277 °C |
| IR (KBr, $cm^{-1}$) | 3374, 1665,1632, 1410 |
| $^1$H NMR (DMSO $d_6$, 400 MHz) ä | 11.88 (2H,s), 9.66 (2H,s), 9.29 - 9.24 (4H,m), 8.48 - 8.44 (2H,m), 8.25 - 8.23 (4H,m), 7.43 - 7.41 (2H,m), 6.53 (4H,s). |
| Mass (m/z) | 491, 492, 493, 494 |

**Example 18**

**1-(2-Phenylamino-2-oxo ethyl)-3-({2-(1-oxo-3-cyclohexyl)-propyl}hydrazino-carbonyl}-pyridinium bromide. (Compound No: 17);**

| | |
|---|---|
| Yield | 25% |
| m.p | 234-236 °C (dec) |
| IR (KBr, $cm^{-1}$) | 1689, 1652 and 1625 |
| $^1H$ NMR (DMSO $d_6$, 400 MHz) δ | 11.11 (1H,s), 10.95 (1H,s), 10.23 (1H,s), 9.56 (1H,s), 9.23 - 9.21 (1H,d), 9.06 - 9.04 (1H,d), 8.38-8.35 (1H,m), 7.62 - 7.60 (2H,d), 7.37 - 7.33 (2H,t), 7.12 - 7.09 (1H,t), 5.75 (2H,s), 2.25 - 2.22 (2H,t), 1.72 - 1.60 (5H,m) 1.49 - 1.43 (2H, m), 1.25 - 1.10 (4H,m), 0.91 - 0.83 (2H,m) |
| Mass (m/z) | 409, 410, 411 and 412 |

**Example 19**

**1-(2-Thien-2'-yl-2-oxoethyl)-3-[2-(benzoyloxy)ethylamino carbonyl]-pyridinium bromide (Compound No:18);**

| | |
|---|---|
| Yield | 40% |
| m.p. | 125-127°C |
| IR (KBr, $cm^{-1}$) | 1710 and 1675 |
| $^1H$ NMR (DMSO $d_6$, 400 MHz) δ | 9.48 (1H,s), 9.43 - 9.41 (1H,t), 9.12 - 9.11 (1H,d), 9.05 - 9.02 (1H,d), 8.40 - 8.36 (1H,m), 8.25 - 8.20 (2H,m), 8.00 - 7.98 (2H,m), 7.68 - 7.64 (1H,m), 7.54-750 (2H,m), 7.42 - 7.40 (1H,m), 6.43 (2H,s), 4.46-4.43 (2H,t), 3.77-3.73 (2H,q) |
| Mass (m/z) | 395, 396, 397 and 398 |

**Example 20**

**1-(4-Ethoxy-2, 4-dioxobutyl)-3-(2-(benzoxyloxy)ethylamino carbonyl)-pyridinium chloride. (Compound No: 19);**

| | |
|---|---|
| Yield | 35% |
| m.p. | 147-149°C |
| IR (KBr, $cm^{-1}$) | 1743, 1720, 1680 and 1627 |
| $^1H$ NMR (DMSO $d_6$, 400 MHz) δ | 9.62 - 9.59 (1H,t), 9.32 - 9.29 (1H,s), 9.05 - 9.03 (1H,d), 8.93 - 8.90 (1H,d), 8.27 - 8.24 (1H,m), 7.92 - 7.89 (2H,d), 7.59 - 7.55 (1H,m), 7.45 - 7.41 (2H,m), 5.82 (2H,s), 4.37-4.34 (2H,t), 4.08-4.03 (2H,q), 3.80 (2H,s), 3.67-3.63 (2H,q), 1.15-1.11 (3H,t), |
| Mass (m/z) | 399, 400 and 401 |

**Example 21**

**1-(2',4'-Dichloro-phenyl-2-oxoethyl)-3-(2-methoxyethyl aminocarbonyl)-pyridinium bromide. (Compound No: 20);**

| | |
|---|---|
| Yield | 70% |
| m.p. | 93-95 °C |
| IR (KBr, $cm^{-1}$) | 1704, 1664 and 1636 |

(continued)

| | |
|---|---|
| $^1H$ NMR (DMSO $d_6$, 400 MHz) δ | 9.48 (1H,s), 9.29 (1H,bs), 9.11 - 9.08 (2H,m), 8.41 - 8.38 (1H,m), 8.15 - 8.13 (1H,d), 7.92 - 7.91 (1H,t), 7.78 - 7.75 (1H,m), 6.44 (2Hs,) 3.52 (2H,bs), 3.51 (2H,bs), 3.28 (3H,s) |
| Mass (m/z) | 367,368,369 and 370 |

**Example 22**

**N,N'-Bis-[3-carbonyl-1-(2-cyclopropylamino-2-oxoethyl) pyridinium] hydrazine dichloride. (Compound No: 21);**

| | |
|---|---|
| Yield | 40% |
| m.p. | 228-230 °C |
| IR (KBr $cm^{-1}$) | 1675,1636 and 1298 |
| $^1H$ NMR (DMSO $d_6$, 400 MHz) δ | 11.85 (2H,s), 9.59 (2H,s), 9.25 - 9.19 (4H,m), 9.00 - 8.99 (2H,d), 8.39 - 8.36 (2H,m), 5.53 (4H,s), 2.73 -2.66 (2H,m), 0.78 - 0.62 (4H,m), 0.53 - 0.49 (4H,m) |
| Mass (m/z) | 437, 438 and 439 |

**Example 23**

**1-(2-Cyclopropylamino-2-oxoethyl)-3-(2-methoxyethylaminocarbonyl)-pyridinium chloride. (Compound No: 22);**

| | |
|---|---|
| Yield | 10% |
| m.p. | 122-124 °C |
| IR (KBr, $cm^{-1}$) | 1661, 1633, 1549 and 1121 |
| $^1H$ NMR (DMSO $d_6$, 400 MHz) δ | 9.40 (1H,s), 9.08 - 9.02 (2H,m), 8.28 - 8.25 (1H,m), 5.53 (2H,s), 3.66 - 3.61 (4H,m), 3.39 (3H,s), 2.78 - 2.74 (1H,m), 0.80 - 0.75 (2H,m), 0.64 - 0.61 (2H,m) |
| Mass (m/z) | 278, 279 and 280 |

**Example 25**

**1-(2-Thien-2'yl-2-oxoethyl)-3-(2-(2-chloro-3-pyridoylhydrazinocarbonyl)-pyridinium chloride. (Compound No: 24);**

| | |
|---|---|
| Yield | 56% |
| m.p. | 233-235 °C |
| IR (KBr, $cm^{-1}$) | 1680,1637,1404 and 1293 |
| $^1H$ NMR (DMSO $d_6$, 400 MHz) δ | 11.62 (1H,s), 11.05 (1H,s), 9.62 (1H,s), 9.24 - 9.23 (1H,d), 9.18 - 9.16 (1H,d), 8.58 - 8.56 (1H,m), 8.46 - 8.43 (1H,m), 8.26 - 8.24 (2H,m), 8.02 - 8.00 (1H,m), 7.61-7.58 (1H,m), 7.43 - 7.41 (1H,m), 6.51 (2H,s) |
| Mass (m/z) | 401, 402, 403, 404 and 405 |

**Example 26**

**1-(2-Isopropylamino-2-oxoethyl)-3-(2-methylsulfonylhydrazinocarbonyl)-pyridinium chloride. (Compound No: 25;**

| | |
|---|---|
| Yield | 10% |
| m.p. | 227 - 229 °C |
| IR (KBr, $cm^{-1}$) | 1691, 1670, 1566 and 1330 |
| $^1H$ NMR (DMSO $d_6$, 400 MHz) δ | 11.55 (1H,s), 9.94 (1H,s), 9.52 (1H,s), 9.16 - 9.14 (1H,m), 9.09 - 9.07 (1H,m), 8.72 - 8.70 (1H,m), 8.34 - 8.30 (1H,m), 5.50 (2H,s), 3.89 - 3.84 (1H,m), 3.11 (3H,s), 1.13 - 1.12 (6H,d) |
| Mass (m/z) | 315, 316 and 317 |

**Example 28**

**1-(2-Thien-2'-yl-2-oxoethyl)-3-(methanesulfonyl hydrazino carbonyl) pyridinium chloride (Compound No: 27);**

| | |
|---|---|
| Yield | 31.00% |
| m.p. | 215- 217°C |
| IR (KBr, $cm^{-1}$) | 1685, 1666 and 1635 |
| $^1HNMR$ :(DMSO $d_6$, 400 $MH_z$) δ | 11.49, (1H, s), 9.96 (1H, s), 9.55 (1H, s), 9.18 (1H, d), 9.10 (1H, d), 8.43 - 8.39 (1H, t), 8.25 - 8.22 (2H, m), 7.42 (1H, t) 6.47 (2H, s), 3.09 (3H, s). |
| Mass (m/z) | 340, 341, 342 and 343 |

**Example 30**

**1-(2-Thien-2'-yl-2-oxoethyl)-3-((2-methoxy ethyl) amino carbonyl)-5-bromo pyridinium chloride (Compound No: 29);**

| | |
|---|---|
| Yield | 31.00% |
| m.p. | 180 - 182°C |
| IR (KBr, $cm^{-1}$) | 1661 and 1620 |
| $^1HNMR$ (DMSO $d_6$, 400 $MH_z$) δ | 9.58 - 9.54 (2H, d), 9.43 - 9.39 (2H, d), 8.25 - 8.21 (2H, m), 7.41 (1H, t), 6.43 (2H, s), 3.51 (4H, m), 3.29 (3H, s). |
| Mass (m/z) | 384, 385, 386, 387 and 388 |

**Example 31**

**1-(2-Thien-2'-yl-2-oxoethyl)-3-[1-oxo-1-(2-methoxycarbonyl) pyridyl] hydrazino pyridinium chloride (Compound No: 30);**

| | |
|---|---|
| Yield | 30.00% |
| m.p. | 222 - 225°C |
| IR (KBr, $cm^{-1}$) | 1726, 1708 and 1662 |
| $^1H$ NMR (DMSO $d_6$, 400 $MH_z$) δ | 11.47 (1H, s), 11.23 (1H, s), 9.58 (1H, s), 9.22 - 9.15 (3H, m), 8.56 - 8.53 (1H, d), 8.46 - 8.43 (1H, t) 8.25 - 8.21 (3H, m), 7.42 (1H, t), 6.49 (2H, s), 3.95 (3H, s) |
| Mass (m/z) | 425, 426 and 427 |

**Example 32**

**1-[1-(2-Thien-2'-yl-2-oxoethyl)-6methyl-3-carbonyl pyridinium]-2-[1-(2-Thien-2'-yl-2-oxoethyl)-3-carbonyl pyridinium] hydrazine dichloride(compound no: 31),**

| | |
|---|---|
| Yield | 40% |
| M.P. | 76-80 °c (dec) |
| IR (KBr,$cm^{-1}$) | 1637,1513 |
| $^1$HNMR (DMSO $d_6$, 400 MHz) δ | 11.69(2H,s), 9.59-9.53(2H,d), 9.19(2H,m), 9.05(1H,d), 8.46-8.43(1H,t) ,8.34 (1H,d), 8.27-8.23(4H,m), 7.45-7.41 (2H,m) ,6.56(2H,s) ,6.48(2H,s) ,2.81(3H,s). |
| Mass(m/z) | 505,506,507. |

**Example 33**

**1-(2-Thien-2'-yl-2-oxoethyl)-3-(isopropylsulfonyl hydrazino carbonyl) pyridinium bromide(compound no: 32),**

| | |
|---|---|
| Yield | 70% |
| M.P | 90-95°c (dec) |
| IR (KBr,$cm^{-1}$) | 1638,1589 |
| $^1$HNMR (DMSO $d_6$, 400 MHz) δ | 11.27(1H,s),9.91(1H,s), 9.60(1H,s) ,9.19-9.15(2H,m), 8.42-8.36(1H,m) , 8.25-8.21(2H,m) ,7.43-7.41(1H,t) ,6.45(2H,s), 1.35-1.34(6H,d). |
| Mass(m/z) | 368,369,370 |

**Example 34**

**1-(2-(4-Benzyl piperidin-1-yl)-2-oxoethyl)-3-(methanesulfonyl hydrazino carbonyl) pyridinium chloride (compound no: 33),**

| | |
|---|---|
| Yield | 17% |
| M.P | 76-78°c |
| IR (KBr,$cm^{-1}$) | 1684,1650,1556,1540. |
| $^1$HNMR (DMSO $d_6$, 400 MHz) δ | 11.46(1H,s) ,9.55(1H,s) ,9.46(1H,s) ,9.09-9.03(2H,m), 8.36-8.32(1H,t), 7.33-7.29(2H,m), 7.23-7.19(3H,m), 5.88-5.79(2H,m) ,4.30-4.27(1H,d) , 3.76-3.73(1H,d), 3.10(4H,m) ,2.64(1H,t) ,2.57-2.55(2H,d), 1.85(1H,bs) , 1.72-1.63(2H,t) ,1.36-1.28(1H,q) ,1.13-1.03(1H,m) |
| Mass(m/z) | 431,432,433 |

**Example 35**

**1-(2-(2-Ethoxy carbonyl pyrrolidin-1-yl)-2-oxoethyl)-3-(methanesulfonyl hydrazino carbonyl) pyridinium chloride. (compound no: 34),**

| | |
|---|---|
| Yield | 14% |
| M.P | 88-91°c |
| IR (KBr,$cm^{-1}$) | 1735,1665,1539 |

(continued)

| | |
|---|---|
| $^1$HNMR (DMSO $d_6$, 400 MHz) δ | 11.48(1H,s) ,9.96(1H,s) ,9.46(1H,s) ,9.09-9.05(2H,m) ,8.38-8.34(1H,t), 5.94-5.80(2H,q) ,4.37-4.36(1H,d), 4.08-4.06(2H,d), 3.68-3.65(2H,m), 3.09(4H, m) ,2.23-2.18(2H,m), 2.04 -1.93(3H,m) ,1.18-1.09(3H,t) |
| Mass(m/z) | 399,400,401 |

**Example 36**

**1-(2-Thien-2'-yl-2-oxoethyl)-3-(methanesulfonyl hydrazino carbonyl)-5-bromo pyridinium bromide. (compound no: 35),**

| | |
|---|---|
| Yield | 54% |
| M.P | Above 190-195°c(dec) |
| IR (KBr,$cm^{-1}$) | 1682,1557,1540,1520 |
| $^1$HNMR (DMSO $d_6$, 400 MHz) δ | 11.35(1H,s) ,10.01(1H,s) ,9.57-9.54(2H,d), 9.32(1H,s) , 8.26-8.22(2H,m), 7.42 (1H,s), 6.39(2H,s), 3.08(3H,s) |
| Mass (m/z) | 418,419,420 |

**Example 37**

**1-(2-Thien-2'-yl-2-oxoethyl)-3-(ethoxycarbonyl hydrazino carbonyl) pyridinium bromide. (compound no: 36),**

| | |
|---|---|
| Yield | 69% |
| M.P | 155-157°c |
| IR (KBr,$cm^{-1}$) | 1731,1665,1637 |
| $^1$HNMR (DMSO $d_6$, 400 MHz) δ | 11.04(1H,s) , 9.59(1H,s) , 9.53(1H,s) , 9.18(1H,s) , 9.05-9.04(1H,d) , 8.42(1H, s) , 8.25-8.23(2H,m) , 7.43(1H,s) , 6.46(2H,s), 4.12-4.11(2H,s), 1.23(3H,s) |
| Mass (m/z) | 334,335,336 |

**Example 38**

**1-(2-(5-chloro-thien-2-yl)-2-oxoethyl)-3-(methanesulfonyl hydrazino carbonyl) pyridinium bromide (compound no: 37),**

| | |
|---|---|
| Yield | 87% |
| M.P | 228-230°c |
| IR (KBr,$cm^{-1}$) | 1708,1664,1631,1550 |
| $^1$HNMR (DMSO $d_6$, 400 MHz) δ | 11.40(1H,s) , 9.98(1H,s) , 9.50(1H,s) , 9.15(1H,d) , 9.061 H,d), 8.43-8.39(1H,t) , 8.16-8.15(1H,d), 7.51-7.50(1H,d) , 6.41(2H,s) , 3.09 (3H,s) |
| Mass (m/z) | 374,375,376,377 |

**Example 39**

**N-N'-Bis[3-carbonyl-1-(2-(4-nitro-thien-2-yl)-2-oxoethyl)pyridinium] hydrazine dichloride. (compound no: 38),**

| | |
|---|---|
| Yield | 27% |

(continued)

| | |
|---|---|
| M.P | 204-207°c |
| IR (KBr,$cm^{-1}$) | 1681,1539,1514 |
| $^1$HNMR (DMSO $d_6$, 400 MHz) δ | 11.90(2H,s) , 9.63(2H,s) , 9.31-9.30(4H,m) , 9.24 -9.22(2H,m), 8.87(2H,s) , 8.49-8.46(2H,t), 6.56 (4H,s) |
| Mass (m/z) | 581,582,583 |

**Example 40**

**1-(2-Thien-2'-yl-2-oxoethyl)-3-(methanesulfonyl hydrazino carbonyl) -6-methyl pyridinium bromide. (compound no: 39),**

| | |
|---|---|
| Yield | 14% |
| M.P | 90-95°c(dec) |
| IR (KBr,$cm^{-1}$) | 1677,1575 |
| $^1$HNMR (DMSO $d_6$, 400 MHz) δ | 11.32(1H,s), 9.97(1H,s) 9.52(1H,s) , 8.94-8.92(1H,d) , 8.32-8.24(3H,m) , 7.44 (1H,t) , 6.54(2H,s), 3.08(3H,s), 2.79(3H,s) |
| Mass (m/z) | 354,355,356 |

**Example 41**

**N-N'-Bis[3-carbonyl-1-(2-(5-methyl-thien-2-yl)-2-oxoethyl) pyridinium] hydrazine dichloride. (compound no: 40),**

| | |
|---|---|
| Yield | 37% |
| M.P | Above 166-168°c(dec) |
| IR (KBr,$cm^{-1}$) | 1666,1500 |
| $^1$HNMR (DMSO $d_6$, 400 MHz) δ | 11.73(2H,s) , 9.59(2H,s) , 9.19-9.15(4H,d) 8.45-8.42(2H,t), 8..06-8.05(2H,d) , 7.15-7.14(2H,d), 6.43 (4H,s), 2.59(6H,s) |
| Mass(m/z) | 519,520,521,522 |

**Example 42**

**N-N'-Bis[3-carbonyl-1-(2-(2-ethoxycarbenyl pyrrolidin-1-yl)-2-oxoethyl) pyridinium] hydrazine dichloride. (compound no: 41),**

| | |
|---|---|
| Yield | 28% |
| M.P | 118-120°c |
| IR (KBr,$cm^{-1}$) | 1660,1510 |
| $^1$HNMR (DMSO $d_6$, 400 MHz) δ | 11.75(2H,s) , 9.51(2H,s), 9.20-9.10(4H,m) 8.43-8.40(2H,t), 5.97-5.83(4H,m) , 4.39-4.36(2H,m), 4.27-4.22(1H,q), 4.12-4.05(4H,m), 3.71-3.63(4H,m), 3.48-3.40(1H,m), 2.26-2.19(2H,m), 2.05-1.91(5H,m) , 1.30-1.27(1H,t), 1.19-1.15(5H,t) |
| Mass(m/z) | 609,610,611 |

**Example 43**

**1-[1-(2-Thien-2'-yl-2-oxoethyl)-5-aminocarbonyl-3-carbonyl pyridinium]-2-[1-(2-Thien-2'-yl-2-oxoethyl)-3-carbonyl pyridinium] hydrazine dichloride (compound no: 42),**

| | |
|---|---|
| Yield | 54% |
| M.P | Above 127-129°c(dec) |
| IR (KBr,$cm^{-1}$) | 1678,1513 |
| $^1$HNMR (DMSO $d_6$, 400 MHz) δ | 11.86(2H,s) , 9.83-9.64(4H,t) , 9.24 -9.23(2H,s) 8.82(1H,s), 8.48-8.45(1H,t), 8.34(1H,s) 8.26-8.24(4H,m), 7.44 -7.42(2H,d), 6.52-6.46(4H,d) |
| Mass (m/z) | 534,535,536 |

**Example 44**

**1-(2-(4-carbethoxy-thiazolidin-3yl)-2-oxoethyl)-3-(methanesulfonyl hydrazino carbonyl) pyridinium chloride (compound no: 43),**

| | |
|---|---|
| Yield | 29% |
| M P | 190-192°c |
| IR (KBr,$cm^{-1}$) | 1673,1541 |
| $^1$HNMR (DMSO $d_6$, 400 MHz) δ | 11.50(1H,s) , 9.55(1H,s) , 9.48(1H,s) , 9.12-9.08(2H,m) , 8.39-8.34(1H,t), 6.04 - 5.99(2H,m) , 4.94 -4.91(1H,m) , 4.87-4.84(1H,d), 4.73-4.71(1H,d), 4.28-4.23 (1H,q), 4.14 -4.09(1H,q), 3.43-3.38(1H,m),3.27-3.22(1H,m), 3.10(3H,s) , 1.30-1.27(1H,t), 1.20-1.17(2H,m) |
| Mass(m/z) | 439,440,441 |

**Example 45**

**N-N'-Bis[3-carbonyl-1-(2-(5-chloro-thien-2-yl)-2-oxoethyl) pyridinium] hydrazine dichloride. (compound no: 44),**

| | |
|---|---|
| Yield | 35% |
| M.P | Above 200-205°c (dec) |
| IR (KBr,$cm^{-1}$) | 1674,1590,1500 |
| $^1$HNMR (DMSO $d_6$, 400 MHz) δ | 11.90(2H,s), 9.64 -9.61(2H,d), 9.29-9.20(4H,m), 8.47-8.44(2H,t), 8.18-8.17(2H, d),7.51-7.50(2H,d), 6.49-6.48(4H,s) |
| Mass(m/z) | 559,560,561,562,563,564 |

**Example 46**

**1-(2-(5-Methyl-thien-2-yl)-2-oxoethyl)-3-(methanesulfonyl hydrazino carbonyl) pyridinium chloride (compound no: 45),**

| | |
|---|---|
| Yield | 22% |
| M.P | 196-198°c |
| IR (KBr,$cm^{-1}$) | 1689,1657 |

(continued)

| | |
|---|---|
| $^1$HNMR (DMSO $d_6$, 400 MHz) δ | 11.47(1H,s) ,9.98(1H,s) ,9.53(1H,s) ,9.17-9.16(1H,d) , 9.09-9.07(1H,d) , 8.42-8.38(1H,t), 8.06-8.05(1H,d) , 7.15-7.14(1H,d), 6.41(2H,s), 3.09(3H,s), 2.59(3H,s) |
| Mass(m/z) | 354,355,356,357 |

**Example 47**

**1-(2-(4-Nitro-thien-2-yl)-2-oxoethyl)-3-(methanesulfonyl hydrazino carbonyl) pyridinium bromide. (compound no: 46),**

| | |
|---|---|
| Yield | 52% |
| M.P | Above 200-205°c(dec) |
| IR (KBr,cm$^{-1}$) | 1688,1631,1541 |
| $^1$HNMR (DMSO $d_6$, 400 MHz) δ | 11.41(1H,s) , 9.50(1H,s) 9.309-9.306(1H,d), 9.17-9.15(1H,d) ,9.09-9.07(1H,d) , 8.866-8.862(1H,d) ,8.45-8.41(1H,t) , 6.50(2H,s), 3.09(3H,s) |
| Mass(m/z) | 385,386,387 |

**Example 48**

**1-(2-Phenylamino-2-oxoethyl)-3-(phenyl hydrazino carbonyl) pyridinium chloride (compound no: 47),**

| | |
|---|---|
| Yield | 45% |
| M.P | 165-167°c |
| IR (KBr,cm$^{-1}$) | 1679,1626,1600,1497 |
| $^1$HNMR (DMSO $d_6$, 400 MHz) δ | 11.18(1H,s) , 11.10(1H,s) , 9.62(1H,s) , 9.24-9.22(1H,d) , 9.17-9.15(1H,d), 8.40-8.36(1H,t) , 8.19(1H,s), 7.63-7.61(2H,d) , 7.37-7.33(2H,t) , 7.20-7.16(2H, t), 7.12-7.09(1H,t) , 6.88-6.86(2H,d), 6.78-6.74(1H,t) , 5.78(2H,s) |
| Mass(m/z) | 347,348,349 |

**Example 49**

**1-(2-Phenylamino-2-oxoethyl)-4 -[2-(benzoyloxy) ethylamino carbonyl] pyridinium chloride (compound no: 48),**

| | |
|---|---|
| Yield | 40% |
| M.P | 178-180°c |
| IR (KBr,cm$^{-1}$) | 1700,1666,1559 |
| $^1$HNMR (DMSO $d_6$, 400 MHz) δ | 11.13(1H,s) , 9.74 -9.71(1H,t) , 9.23-9.22(2H,d) , 8.52-8.50(2H,d) , 8.01-7.99 (2H,d) , 7.68-7.60(3H,m) , 7.54-7.51(2H,t) , 7.36-7.32(2H,t) , 7.12-7.08 (1H,t) , 5.75 (2H,s) , 4.47-4.45(2H,t) , 3.77-3.72(2Hq). |
| Mass (m/z) | 404,405,406 |

**Example 50**

**1-2-(5-Nitro-thien-2-yl)-2-oxoethyl)-3-(methanesulfonyl hydrazino carbonyl) pyridinium chloride (compound no: 49),**

| | |
|---|---|
| Yield | 10% |
| M.P | Above 105-110°c(dec) |
| IR (KBr,$cm^{-1}$) | 1680,1620 |
| $^1$HNMR (DMSO $d_6$, 400 MHz) δ | 11.48(1H,s), 9.98(1H,s), 9.52(1H,s) , 9.16-9.10(2H,m) ,8.45-8.41(1H,t) , 8.35-8.34(1H,d) ,8.25-8.24(1H,d) , 6.50(2H,s), 3.09(3H,s). |
| Mass (m/z) | 385,386,387 |

**Example 51**

**1-(2-Thien-2'-yl-2-oxoethyl)3-(Trifluromethanesulfonyl hydrazino carbonyl)-pyridinium bromide (compound no: 50),**

| | |
|---|---|
| Yield | 22% |
| M.P | 77-79°c |
| IR (KBr,$cm^{-1}$) | 2960, 1690, 1673, 1591 |
| $^1$HNMR (DMSO $d_6$, 400 MHz) δ | 11.76(1H,s) , 11.27(1H,s), 9.61(1H,s) , 9.20-9.19(1H,d) ,9.07-9.05(1H,d) , 8.44-8.41(1H,t) ,8.25-8.22(2H,m) , 7.34 - 7.41 (1H,m), 6.46 (2H,s). |
| Mass (m/z) | 394, 395, 396 |

**Example 52**

**1-(2-Thien-2'-yl-2-oxoethyl)-3-(phenyl hydrazino carbonyl) pyridinium bromide (compound no. 51),**

| | |
|---|---|
| Yield | 10% |
| M.P | 192-194°c |
| IR (KBr,$cm^{-1}$) | 1669,1663,1603, |
| $^1$HNMR (DMSO $d_6$, 400 MHz) δ | 10.99(1H,s) , 9.54(1H,s) , 9.17-9.14(2H,t), 8.44-8.41(1H,t) , 8.25-8.22(3H,m), 7.43-7.41(1H,t) , 7.20-7.16(2H,t), 6.87-6.85(2H,d) , 6.79-6.75(1H,t) , 6.46(2H,s) |
| Mass(m/z) | 338,339,340 |

**Example 53**

**1-(2-Thien-2'-yl-2-oxoethyl)-3-(p-methoxy phenyl sulfonyl hydrazino carbonyl) pyridinium bromide (compound no. 52),**

| | |
|---|---|
| Yield | 28% |
| M.P | 126-128°c |
| IR (KBr,$cm^{-1}$) | 1672,1653,1596 |
| $^1$HNMR (DMSO $d_6$, 400 MHz) δ | 11.34 -11.33(1H,d), 10.27-10.26(1H,d), 9.34(1H,s), 9.13-9.12(1H,d) , 8.94-8.92 (1H,d), 8.38-8.34(1H,t) ,8.24-8.19(2H,m),7.82-7.75(2H,m) ,7.42-7.40(1H,t), 7.07-7.04(2H,d) ,6.40(2H,s), 3.81(3H,s). |
| Mass(m/z) | 432,433,434 |

**Example 54**

**1-(2-Ethoxy-2-oxoethyl)-3-(phenyl aminocarbonyl hydrazino carbonyl) pyridinium bromide. (compound no. 53),**

| | |
|---|---|
| Yield | 25% |
| M.P | 183-185°c |
| IR ($KBr, cm^{-1}$) | 1746,1717,1682 |
| $^1HNMR$ (DMSO $d_6$, 400 MHz) $\delta$ | 11.02(1H,s) , 9.57(1H,s) , 9.22-9.21(1H,d), 9.11-9.09(1H,d) , 9.00(1H,s), 8.57 (1H,s) ,8.44-8.41(1H,m),7.47-7.45(2H,d) ,7.29-7.25(2H,t) , 7.00-6.96(1H,t), 5.74(2H,s), 4.28-4.23(2H,q), 1.28-1.25(3H,t). |
| Mass(m/z) | 343,344,345,346 |

**Example 55**

**1-(2-Ethoxy-2-oxoethyl)-3-(p-toluene sulfonyl hydrazino carbonyl) pyridinium bromide. (compound no. 54),**

| | |
|---|---|
| Yield | 54% |
| M.P | 174-176°c |
| IR ($KBr, cm^{-1}$) | 1746,1712,1634 |
| $^1HNMR$ (DMSO $d_6$, 400 MHz) $\delta$ | 11.33(1H,s) , 10.36(1H,s) , 9.37(1H,s) , 9.18-9.16(1H,d) , 8.93-8.91(1H,d) , 8.37-8.33(1H,t) , 7.78-7.76(2H,d) , 7.37-7.35(2H,d) , 5.68 (2H,s) , 4.26-4.20 (2H,q) ,2.37(3H,s),1.27-1.23(3H,t). |
| Mass (m/z) | 378,379,380,381 |

**Example 56**

**1-(2-Phenyl-2-oxoethyl)-3-(phenylamino carbonyl hydrazino carbonyl) pyridinium bromide. (compound no. 55),**

| | |
|---|---|
| Yield | 70% |
| M.P | 206-208°c |
| IR ($KBr, cm^{-1}$) | 1713,1684,1634 |
| $^1HNMR$ (DMSO $d_6$ 400 MHz) $\delta$ | 11.05(1H,s), 9.55(1H,s) , 9.18-9.13(2H,m) , 9.02(1H,s) , 8.59(1H,s) , 8.49-8.45 (1H,m) ,8.09-8.07(2H,d), 7.84-7.80(1H,t), 7.71-7.67(2H,t) , 7.49-7.47(2H,d), 7.30-7.26(2H,t) , 7.01-6.97 (1H,t) ,6.56(2H,s) . |
| Mass (m/z) | 375,376,377 |

**Example 57**

**1-(2-Phenylamino-2-oxoethyl)-3-(benzyl sulfonyl hydrazino carbonyl) pyridiniumchloride. (compound no. 56),**

| | |
|---|---|
| Yield | 48% |
| M.P | 208-210°c |
| IR ($KBr, cm^{-1}$) | 1712,1681,1632 |
| $^1HNMR$ (DMSO $d_6$, 400 MHz) $\delta$ | 11.46(1H,s),10.80(1H,s) , 9.59(1H,s), 9.22-9.20(1H,d) ,9.08-9.06(1H,d), 8.38-8.36(1H,t),7.60-7.58(2H,d) ,7.49(2H,m) , 7.39-7.34(5H,m),7.13-7.10(1H, t), 5.74(2H,s), 4.52(2H,s),. |
| Mass(m/z) | 425,426,427,428 |

**Example 58**

**1-(2-Phenyl-2-oxoethyl)-4-(methanesulfonyl hydrazino carbonyl) pyridinium bromide (compound no. 57),**

| | |
|---|---|
| Yield | 10% |
| M.P | 190-192°c |
| IR (KBr,$cm^{-1}$) | 1679,1630,1650 |
| $^1$HNMR (DMSO $d_6$, 400 MHz) δ | 11.54(1H,s) , 10.03(1H,s), 9.20-9.18(2H,d), 8.59-8.57(2H,d), 8.10-8.08(2H,d) , 7.84 -7.80(1H,t), 7.71-7.67(2H,t) , 6.56(2H,s) , 3.08(3H,s). |
| Mass(m/z) | 334,335,336 |

**Example 59**

**1-(2-Phenyl-2-oxoethyl)-3-(phenyl hudrazino carbonyl) pyridinium bromide (compound no. 58),**

| | |
|---|---|
| Yield | 36% |
| M.P | 204-206°c |
| IR (KBr,$cm^{-1}$) | 1686,1653,1630 |
| $^1$HNMR (DMSO $d_6$, 400 MHz) δ | 11.01(1H,s) ,9.53(1H,s) ,9.17-9.16(2H,m) , 8.46-8.42(1H,t) , 8.09-8.07(2H,d) , 7.82-7.78(1H,t), 7.69-7.65(2H,t), 7.20-7.16(2H,t), 6.88-6.86(2H,d), 6.79-6.75 (1H,t), 6.56(2H,s) |
| Mass(m/z) | 332,333 |

**Example 60**

**1-(2-Ethoxy-2-oxoethyl)-4-[2-(benzoyloxy) ethyl amino carbonyl] pyridinium bromide (compound no. 59),**

| | |
|---|---|
| Yield | 82% |
| M.P | 154-156°c |
| IR (KBr,$cm^{-1}$) | 1742,1719,1707,1675 |
| $^1$HNMR (DMSO $d_6$, 400 MHz) δ | 9.57-9.54(1H,t), 9.22-9.20(2H,d) , 8.51-8.49(2H,d), 8.00-7.98(2H,d), 7.68-7.64 (1H,t), 7.54 -7.51(2H,t), 5.72(2H,s), 4.47-4.44(2H,t) , 4.27-4.21 (2H,q) , 3.76-3.72(2H,q), 1.27-1.24. (3H,t) |
| Mass(m/z) | 357,358,359. |

**Example 61**

**1-(2-Ethoxy-2-oxoethyl)-3-(phenyl hydrazino carbonyl) pyridinium bromide.(compound no. 60),**

| | |
|---|---|
| Yield | 37% |
| M.P | 185-187°c |
| IR (KBr,$cm^{-1}$) | 1740,1690,1630. |
| $^1$HNMR (DMSO $d_6$, 400 MHz) δ | 11.01(1H,s) , 9.58(1H,s) , 9.23-9.14(2H,m), 8.42-8.39(1H,t), 8.19(1H,s) , 7.20-7.16(2H,t), 6.87-6.85(2H,d), 6.78-6.75(1H,t) , 5.75(2H,s), 4.28-4.22(2H, q), 1.28-1.24(3H,t) |
| Mass(m/z) | 300,301,302. |

**Example 62**

**1-(2-Phenyl-2-oxoethyl)-3-(p-methoxyphenyl sulfonyl hydrazino carbonyl) pyridinium bromide (compound no. 61),**

| | |
|---|---|
| Yield | 59% |
| M.P | 188-190°c |
| IR (KBr,$cm^{-1}$) | 1671,1634,1580. |
| $^1$HNMR (DMSO $d_6$, 400 MHz) δ | 11.26-11.25(1H,d), 10.17- 10.16(1H,d), 9.24(1H,s), 9.03-9.01(1H,d), 8.87-8.85 (1H,d), 8.31-8.27(1H,t), 7.97-7.96(2H,d), 7.74 -7.69(3H,m), 7.60-7.56(2H,t) , 6.99-6.97(2H,d) , 6.40(2H,s), 3.73(3H,s). |
| Mass(m/z) | 426,427,428,429 |

**Example 63**

**1-(2-Phenyl-2-oxoethyl)- 4-[2-(benzoyloxy) ethyl amino carbonyl] pyridinium bromide (compound no. 62),**

| | |
|---|---|
| Yield | 92% |
| M.P | 202-204°c |
| IR (KBr,$cm^{-1}$) | 1715,1692,1650 |
| $^1$HNMR (DMSO $d_6$, 400 MHz) δ | 9.55(1H,s) , 9.14 -9.13(2H,d) , 8.52-8.51(2H,d), 8.07-7.99(4H,m) , 7.80-7.51 (6H,m) , 6.52(2H,s), 4.46(2H,s), 3.76-3.75(2H,s). |
| Mass(m/z) | 3.89,390,391,392 |

**Example 64**

**1-(2-Ethoxy-2-oxoethyl)- 4-(p-methanesulfonyl hydrazino carbonyl) pyridinium bromide. (compound no. 63),**

| | |
|---|---|
| Yield | 45% |
| M.P | 94-96°c |
| IR (KBr,$cm^{-1}$) | 1726,1681,1643 |
| $^1$HNMR (DMSO $d_6$, 400 MHz) δ | 11.49(1H,s) ,9.98(1H,s) ,9.23-9.21(2H,d), 8.54-8.52(2H,d) , 5.73(2H,s), 4.28-4.22(2H,q), 3.09(3H,s), 1.28-1.25(3H,t). |
| Mass(m/z) | 302,303,304,305. |

**Pharmaceutical Compositions**

Pharmaceutical compositions may be prepared with a pharmaceutically effective quantity of compounds of general formula I, individually or in combination. The following pharmaceutical formulations suggested are by way of example alone and in no way restrict the forms in which they can be used.

**Oral formulations**

Oral formulations may be administered as solid dosage forms for example pellets, powders, sachets or discreet units such as tablets or capsules and like. Other orally administered pharmaceutical preparations include monophasic and biphasic liquid dosage forms either in ready to use form or forms suitable for reconstitution such as mixtures, syrups, suspensions or emulsions. The preparations in addition may contain diluents, dispersing agents, buffers, stabilizers, solubilizers, surfactants, preservatives, chelating agents and/ or other pharmaceutical additives as are used. Aqueous or non aqueous vehicle or their combination may be used and if desired may contain suitable

sweetener, flavoring agent or similar substances. In case of suspension or emulsion a suitable thickening agent or suspending agent or emulsifying agent may be present in addition. Alternatively, the compounds may be administered as such in their pure form unassociated with other additives for example as capsules or sachets. It may also be administered with a vehicle. Pharmaceutical preparations can have a slow, delayed or controlled release of active ingredients as is provided by a matrix or diffusion controlled system.

When the present invention or its salts or suitable complexes is presented as a discreet unit dosage form like tablet, it may contain in addition medically inert excipients as are used in the art. Diluents such as starch, lactose, dicalcium phosphate, talc, magnesium stearate, polymeric substances like methyl cellulose, fatty acids and derivatives, sodium starch glycollate, etc. may also be used.

**Example 65**

**Preparation of oral dosage form:**

A typical tablet has the following composition:

| Active ingredient of formula I | as given above |
|---|---|
| Lactose | 135 mg |
| Starch | 76 mg |
| Polyvinyl pyrolidone (K-30) | 2 mg |
| Talc | 1.5 mg |
| Magnesium Stearate | 1.0 mg |

**Parenteral Formulations**

For parenteral administration, the compounds or their salts or suitable complexes thereof may be present in a sterile vehicle which may be an aqueous or non aqueous vehicle or a combination thereof. The examples of vehicles are water, ethyl oleate, oils and derivatives of polyols, glycols and their derivatives. It may contain additives common in injectable preparations like stabilizers, solubilizers, pH modifiers, buffers, antioxidants, cosolvents, complexing agents, tonicity modifiers, etc.

Some suitable additives are for example tartrate, citrate or similar buffers, alcohol, sodium chloride, dextrose and high molecular weight polymers. Another alternative is sterile powder reconstitution. The compound may be administered in the form of injection for more than once daily administration, or intravenous infusion/ drip or suitable depot preparation.

**Example 66**

**Preparation suitable for parenteral administration has the following composition:**

| Active ingredient of formula I | as given above |
|---|---|
| Polyethylene glycol (400) | 0.75 ml |
| Sodium metabisulphite | 0.01% |
| Isotonic saline/ WFI | q.s. |

**Other Formulations.**

For the dermatological application and for the discoloration of teeth, the recommended formulations are lotions, oral rinse and toothpaste containing appropriate amount of the present specific compounds of the general formula I.

## Claims

**1.** A pyridinium derivative which is selected from the group consisting of the following compounds:

(a) N,N'bis [3-carbonyl-1-(2-thien-2'-yl-2-oxoethyl)pyridinium] hydrazine dichloride,

(b) N,N'bis[3-carbonyl-1-(2-cyclopropylaminio-2-oxoethyl) pyridinium] hydrazine dichloride,

(c) 1-(2-phenylamino-2-oxoethyl)-4-(phenylsulfonyl hydrazino carbonyl)pyridimum chloride and pharmaceutically acceptable salts thereof,

(d) 1-(2-(2',4'-dichlorophenyl)-2-oxoethyl)-3- (2(methoxy) ethyloxycarbonyl) pyridinium bromide and pharmaceutically acceptable salts thereof,

(e) 1-(2-phenylamino-2-oxoethyl)-3-((benzoyloxy) ethylaminocarbonyl) pyridinium chloride and pharmaceutically acceptable salts thereof,

(f) 1-(2-thien-2'-yl-2-oxoethyl)-3-(phenylaminocarbonyl hydrazinocarbonyl) pyridinium bromide and pharmaceutically acceptable salts thereof,

(g) 1-(2-phenyl-2-oxoethyl)-3-(2-(acetoxy) ethylaminocarbonyl) pyridinium bromide and pharmaceutically acceptable salts thereof,

(h) 1-(2-phenylamino-2-oxoethyl)-3-(phenyl sulfonyl hydrazino carbonyl) pyridinium chloride and pharmaceutically acceptable salts thereof,

(i) 1-(2-phenylamino-2-oxoethyl)-3-((4-methylphenyl)sulfonyl hydrazinocarbonyl) pyridinium chloride and pharmaceutically acceptable salts thereof,

(j) 1-(2-phenyl-2-oxoethyl)-3-(2-(benzoyloxy)ethyloxy carbonyl) pyridinium bromide and pharmaceutically acceptable salts thereof,

(k) 1-(2-thien-2'-yl-2-oxoethyl)-3-(phenylcarbonyl hydrazino carbonyl) pyridinium bromide and pharmaceutically acceptable salts thereof,

(l) 1-(2-ethoxy-2-oxoethyl)-3-((phenylmethyl)sulfonyl hydrazino carbonyl)pyridinium bromide and pharmaceutically acceptable salts thereof,

(m) 1-(2-phenyl-2-oxoethyl)-3-((phenylmethyl)sulfonyl hydrazino carbonyl) pyridinium bromide and pharmaceutically acceptable salts thereof,

(n) N,N' bis [3-carbonyl-1-(2-furan-2'-yl-2-oxoethyl)pyridinium] hydrazine dibromide,

(o) 1-(2',4'-dichloro-phenyl-2-oxoethyl)-3-(2-methoxy ethyl aminocarbonyl)-pyridinium bromide,

(p) 1-(2-thien-2'-yl-2-oxoethyl)-3-((2-methoxy ethyl) amino carbonyl)-5-bromo pyridinium chloride,

(q) 1-(2-thien-2'-yl-2-oxoethyl)-3-(methanesulfonyl hydrazino carbonyl) pyridinium chloride,

(r) 1-(2-thien-2'-yl-2-oxoethyl)-3-(2-(2-chloro-3-pyridoylhydrazinocarbonyl)-pyridinium chloride,

(s) 1-(2-cyclopropylamino-2-oxoedtyl)-3-(2-memoxyethylaminocarbonyl)-pyridinium chloride,

(t) 1-(2-isopnopylamino-2-oxoethyl)-3-(2-methylsulfonylhydrazinocarbonyl)-pyridinium chloride,

(u) 1-(2-phenylamino-2-oxoethyl)-3-((2-(1-oxo-3-cyclohexyl)-propyl) hydrazino-carbonyl)-pyridinium bromide,

(v) 1-(2-thien-2'-yl-2-oxoethyl)-3-[2-(benzoyloxy)ethylamino carbonyl]-pyridinium bromide,

(w) 1-(4-ethoxy-2,4-dioxobutyl)-3-(2-(benzoxyloxy)ethylamino carbonyl)-pyridinium chloride,

(x) 1-(2-thien-2'-yl-2-oxoethyl)-3-[1-oxo-1 -(2-methoxy carbonyl) pyridyl] hydrazino pyridinium chloride,

(y) 1-[1-(2-thien-2'-yl-2-oxoethyl)-5-aminocarbonyl-3-carbonyl pyridinium]-2-[1-(2-thien-2'-yl-2-oxoethyl)-3-carbonyl pyridinium] hydrazine dichloride,

(z) 1-(2-thien-2'-yl-2-oxoethyl)-3-(trifluoromethanesulfonyl hydrazino carbonyl) - pyridinium bromide,

(aa)1-[1-(2-thien-2'-yl-2-oxoethyl)-6-methyl-3-carbonylpyridinium]-2-[1-(2-thien-2'-yl-2-oxoethyl)-3-carbonyl pyridinium] hydrazine dichloride,

(ab) N,N'-bis[3-carbonyl-1-(2-(5-methyl-thien-2-yl)-2-oxoethyl) pyridinium] hydrazine dichloride,

(ac) N,N'-bis[3-carbonyl-1-(2-(5-chloro-thien-2-yl)-2-oxoethyl) pyridinium] hydrazine dichloride,

(ad) 1-(2-thien-2'-yl-2-oxoethyl)-3-(methanesulfonyl hydrazino carbonyl)-6-methyl pyridinium bromide,

(ae) N,N'-bis[3-carbonyl-1 -(2-(4-nitro-thien-2-yl)-2-oxoethyl) pyridinium] hydrazine dichloride,

(af) 1-(2-phenylamino-2-oxoethyl)-3-(phenyl hydrazino carbonyl) pyridinium chloride,

(ag) 1-(2-(4-nitro-thien-2-yl)-2-oxoethyl)-3-(methanesulfonyl hydrazino carbonyl) pyridinium bromide,

(ah) 1-(2-(5-nitro-thien-2-yl)-2-oxoethyl)-3-(methanesulfonyl hydrazino carbonyl) pyridinium chloride,

(ai) 1-(2-(5-chloro-thien-2-yl)-2-oxoethyl)-3-(methanesulfonyl hydrazino carbonyl) pyridinium bromide,

(aj) 1-(2-thien-2'-yl-2-oxoethyl)-3-(ethoxycarbonyl hydrazino carbonyl) pyridmium bromide,

(ak) 1-(2-thien-2'-yl-2-oxoethyl)-3-(isopropylsulfonyl hydrazino carbonyl) pyridinium bromide,

(al) 1-(2-thien-2'-yl-2-oxoethyl)-3-(methanesulfonyl hydrazino carbonyl)-5-bromo pyridinium bromide,

(am) 1-(2-(2-ethoxy carbonyl pyrrolidin-1-yl)-2-oxoethyl)-3-(methanesulfonyl hydrazino carbonyl) pyridinium chloride,

(an) 1-(2-(5-methyl-thien-2-yl)-2-oxoethyl)-3-(methanesulfonyl hydrazino carbonyl) pyridinium chloride,

(ao) 1-(2-(4-carbethoxy-thiazolidin-3-yl)-2-oxoethyl)-3-(methanesulfonyl hydrazino carbonyl) pyridinium chloride,

(ap) 1-(2-(4-benzylpiperidin-1-yl)-2-oxoethyl)-3-(medmesulfonyl hydrazino carbonyl) pyridinium chloride,

(aq) N,N'-bis[3-carbonyl-1-(2-(2-ethoxycarbonyl pyrrolidin-1-yl)-2-oxoethyl) pyridinium] hydrazine dichloride,

(ar) 1-(2-phenylamino-2-oxoethyl)-4-[2-(benzoyloxy) ethylamino carbonyl] pyridinium chloride,

(as) 1-(2-thien-2'-yl-2-oxoethyl)-3-(phenyl hydrazino carbonyl) pyridinium bromide,

(at) 1-(2-thien-2'-yl-2-oxoethyl)-3-(p-methoxy phenyl sulfonyl hydrazino carbonyl) pyridinium bromide,

(au) 1-(2-ethoxy-2-oxoethyl)-3-(phenyl aminocarbonyl hydrazino carbonyl) pyridinium bromide,

(av) 1-(2-ethoxy-2-oxoethyl)-3-(p-toluene sulfonyl hydrazino carbonyl) pyridinium bromide,

(aw) 1-(2-phenyl-2-oxoethyl)-3-(phenylamino carbonyl hydrazino carbonyl) pyridinium bromide,

(ax) 1-(2-phenylamino-2-oxoethyl)-3-(benzyl sulfonyl hydrazino carbonyl) pyridiniumchloride,

(ay) 1-(2-phenyl-2-oxoethyl)-4-(methanesulfonyl hydrazino carbonyl) pyridinium bromide,

(az) 1-(2-phenyl-2-oxoethyl)-3-(phenyl hydrazino carbonyl) pyridinium bromide,

(ba) 1-(2-ethoxy-2-oxoethyl)-4-[2-(benzoyloxy) ethyl amino carbonyl] pyridinium bromide,

(bb) 1-(2-ethoxy-2-oxoethyl)-3-(phenyl hydrazino carbonyl) pyridinium bromide,

(be) 1-(2-phenyl-2-oxoethyl)-3-(p-methoxyphenyl sulfonyl hydrazino carbonyl) pyridinium bromide,

(bd) 1-(2-phenyl-2-oxoethyl)- 4-[2-(benzoyloxy) ethyl amino carbonyl] pyridinium bromide and

(be) 1-(2-ethoxy-2-oxoethyl)- 4-(p-methanesulfonyl hydrazino carbonyl) pyridinium bromide.

**2.** A process for the preparation of compounds as claimed in claim 1, which comprises

i) refluxing a pyridine derivative selected from :

(a) N,N'-bis(nicotinyl)hydrazine
(b) 3-[(2-pyridyl)hydrazinocarbonyl]pyridine
(c) 3-[(2-methanesulfonyl)hydrazinocarbonyl]pyridine
(d) 3-[(2-benzoyloxy)ethylaminocarbonyl]pyridine
(e) 3-[(2-phenylsulfonyl)hydrazinocarbonyl]pyridine
(f) 3-[(2-acetoxy)ethyloxycarbonyl]pyridine
(g) 3-[(2-benzoyloxy)ethyloxycarbonyl]pyridine
(h) 3-[(2-methoxy)ethyloxycarbonyl]pyridine
(i) 3-[(2-phenylaminocarbonyl)hydrazinocarbonyl]pyridine
(j) 3-[(2-acetoxy)ethylaminocarbonyl]pyridine
(k) 3-[(2-(4-methylphenyl sulfonyl)hydrazinocarbonyl]pyridine
(l) 3-[(2-benzoyl)- hydrazinocarbonyl]pyridine
(m) 3-[(2-phenylmethanesulfonyl) hydrazinocarbonyl] pyridine
(n) 3-[(2-(3-cyclohexylpropanoyl)hydrazinocarbonyl]pyridine
(o) 3-[(2-methoxy) ethylaminocarbonyl]pyridine
(p) 3-[1-oxo-1-(2-methoxycarbonyl)pyridyl]hydrazino pyridine

with a quaternizing agent selected from the group comprising of :

(a) 2-bromoacetyl thiophene
(b) 2-chloroacetyl thiophene
(c) phenacylbromide
(d) phenacylchloride
(e) 2,4-dichlorophenacylbromide
(f) N-phenyl chloroacetamide
(g) N-cyclopropyl chloroacetamide
(h) Ethylbromoacetate
(i) Bromo acetylfuran
(j) N-isopropylchloroacetamide
(k) N-chloroacetyl-2-pyrrolidinone and
(l) chloroacetic acid

in an alcoholic solvent selected from the group comprising of:

(a) methanol
(b) ethanol
(c) n-propanol and
(d) isopropanol

and / or a high boiling solvent selected from the group comprising of:

(a) toluene
(b) o-xylene
(c) m-xylene
(d) p-xylene and
(e) xylenes

for 6-48 hours.

(ii) isolating the compound from step (i).

**3.** The use of compound as claimed in claim 1, in the manufacture of a medicament for diabetic complications and aging-related diseases, including kidney disease, nerve damage, retinopathy, atherosclerosis, microangiopathy, endothelial dysfunctions, dermatological conditions, discoloration of teeth and other organ dysfunctions.

**4.** A pharmaceutical composition for treatment of diabetic complications and aging related diseases which comprises a pharmaceutically effective amount of one or more compounds as claimed in claim 1, or pharmaceutically acceptable salt(s) thereof in admixture with a pharmaceutically acceptable carrier, diluent, solvent or excepient.

**5.** The pharmaceutical composition as claimed in claim 4, in the form of an oral formulation.

**6.** The pharmaceutical composition as claimed in claim 5, wherein said pharmaceutically acceptable carrier is selected from group consisting of starch, lactose, polyvinyl pyrolidone (K-30), talc and magnesium stearate.

**7.** The pharmaceutical composition as claimed in claim 4 in the form of a parenteral formulation.

**8.** A method for the preparation of a parenteral formulation as claimed in claim 7, which comprises dissolving one or more compounds as defined in claim 1, in polyethylene glycol 400 and diluting the solution so obtained, with an isotonic solution or water to a desired concentration.

**9.** Pharmaceutical composition as claimed in claim 6, in the form of a lotion, oral rinse and toothpaste.

**10.** Use of the compound of claim 1 for the manufacture of a medicament for treating a diabetic patient by breaking a preformed AGE, within said patient, which comprises, administering an effective amount of said compound, either singly, or in combination with other drugs for antidiabetic therapy.

**11.** Use of the compound of claim 1 for the manufacture of a medicament for preventing or treating diseases caused by diabetes and aging related complications, which comprises, administering to a patient in need thereof, an effective amount of said compound, either singly or in combination with a pharmaceutically acceptable carrier, diluent or excepient.

**12.** The use as claimed in claim 11, wherein the diseaseprevented or treated is a nephrological disorder, neurological disorder, atherosclerosis, retinal disorder, dermatological disorder, non-enzymatic browning of oral cavity, endothelial or other organ dysfunction and growth impairment.

**13.** The use of claim 12, wherein said
compound is selected from the group consisting of:

(a) N,N'bis [3-carbonyl-1-(2-thien-2'-yl-2-oxoethyl)pyridinium] hydrazine dichloride,

(b) N,N'-bis-[3-carbonyl-1-(2-cyclopropylamino-2-oxoethyl) pyridinium] hydrazine dichloride,

(c) 1-(2-phenylamino-2-oxoethyl)-4-(phenylsulfonyl hydrazino carbonyl)pyridinium chloride and pharmaceutically acceptable salts thereof,

(d) 1-(2-(2',4'-dichlorophenyl)-2-oxoethyl)-3-(2(methoxy) ethyloxycarbonyl) pyridinium bromide and phannaceutically acceptable salts thereof,

(e) 1-(2-phenylamino-2-oxoethyl)-3-((benzoyloxy) ethylaminocarbonyl) pyridinium chloride and pharmaceutically acceptable salts thereof,

(f) 1-(2-thien-2'-yl-2-oxoethyl)-3-(phenylaminocarbonyl hydrazinocarbonyl)pyridinium bromide and pharmaceutically acceptable salts thereof,

(g) 1-(2-phenyl-2-oxoethyl)-3-(2-(acetoxy) ethylaminocarbonyl) pyridinium bromide and pharmaceutically acceptable salts thereof,

(h) 1-(2-phenylamino-2-oxoethyl)-3-(phenyl sulfonyl hydrazino carbonyl) pyridinium chloride and pharmaceutically acceptable salts thereof,

(i) 1-(2-phenylamino-2-oxoethyl)-3-((4-methylphenyl) sulfonyl hydrazinocarbonyl) pyridinium chloride and pharmaceutically acceptable salts thereof,

(j) 1-(2-phenyl-2-oxoethyl)-3-(2-(benzoyloxy)ethyloxy carbonyl) pyridinium bromide and pharmaceutically acceptable salts thereof,

(k) 1-(2-thien-2'-yl-2-oxoethyl)-3-(phenylcarbonyl hydrazino carbonyl) pyridinium bromide and pharmaceutically acceptable salts thereof,

(l) 1-(2-ethoxy-2-oxoethyl)-3-((phenylmethyl)sulfonyl hydrazino carbonyl)pyridinium bromide and pharmaceutically acceptable salts thereof,

(m) 1-(2-phenyl-2-oxoethyl)-3-((phenylmethyl)sulfonyl hydrazino carbonyl)pyridinium bromide and pharmaceutically acceptable salts thereof,

(n) N,N-bis[3-carbonyl-1-(2-furan-2'-yl-2-oxoethyl) pyridinium] hydrazine dibromide,

(o) 1-(2',4'-dichloro-phenyl-2-oxoethyl)-3-(2-methoxyethyl aminocarbonyl)-pyridinium bromide,

(p) 1-(2-thien-2'-yl-2-oxoethyl)-3-((2-methoxy ethyl) amino carbonyl)-5-bromo pyridinium chloride,

(q) 1-(2-thien-2'-yl-2-oxoethyl)-3-(methanesulfonyl hydrazino carbonyl) pyridinium chloride,

(r) 1-(2-thien-2'-yl-2-oxoethyl)-3-(2-(2-chloro-3-pyridoylhydrazinocarbonyl)-pyridinium chloride,

(s) 1-(2-cyclopropylamino-2-oxoethyl)-3-(2-methoxyethylaminocarbonyl)-pyridinium chloride,

(t) 1-(2-isopropylamino-2-oxoethyl)-3-(2-methylsulfonylhydrazinocarbonyl)-pyridinium chloride,

(u) 1-(2-phenylamino-2-oxo ethyl)-3-({2-(1-oxo-3-cyclohexyl)-propyl} -hydrazino-carbonyl)-pyridinium bromide,

(v) 1-(2-thien-2'-yl-2-oxoethyl)-3-[2-(benzoyloxy)ethylamino carbonyl]-pyridinium bromide,

(w) 1-(4-ethoxy-2, 4-dioxobutyl)-3-(2-(benzoxyloxy)ethylamino carbonyl)-pyridinium chloride,

(x) 1-(2-thien-2'-yl-2-oxoethyl)-3 -[1-oxo-1-(2-methoxy carbonyl) pyridyl] hydrazino pyridinium chloride,

(y) 1-[1-(2-thien-2'-yl-2-oxoethyl)-5-aminocarbonyl-3-carbonyl pyridinium]-2-[1-(2-thien-2'-yl-2-oxoethyl)-3-carbonyl pyridinium] hydrazine dichloride,

(z) 1-(2-thien-2'-yl-2-oxoethyl)-3-(trifluoromethanesulfonyl hydrazino carbonyl) - pyridinium bromide,

(aa) 1-[1-(2-thien-2'-yl-2-oxoethyl)-6-methyl-3-carbonylpyridinium]-2-[1-(2-thien-2'-yl-2-oxoethyl)-3-carbonyl pyridinium]hydrazine dichloride,

(ab) N,N'-bis[3-carbonyl-1-(2-(5-methyl-thien-2-yl)-2-oxoethyl) pyridinium] hydrazine dichloride,

(ac) N,N'-bis[3-carbonyl-1-(2-(5-chloro-thien-2-yl)-2-oxoethyl) pyridinium] hydrazine dichloride,

(ad) 1-(2-tm'en-2'-yl-2-oxoethyl)-3-(methanesulfonyl hydrazino carbonyl)-6-methyl pyridinium bromide,

(ae) N,N'-bis[3-carbonyl-1 -(2-(4-nitro-thien-2-yl)-2-oxoethyl)pyridinium] hydrazine dichloride,

(at) 1-(2-phenylamino-2-oxoethyl)-3-(phenyl hydrazino carbonyl) pyridinium chloride,

(ag) 1-(2-(4-nitro-thien-2-yl)-2-oxoethyl)-3-(methanesulfonyl hydrazino carbonyl) pyridinium bromide,

(ah) 1-(2-(5-nitro-thien-2-yl)-2-oxoethyl)-3-(methanesulfonyl hydrazino carbonyl) pyridinium chloride,

(ai) 1-(2-(5-chloro-thien-2-yl)-2-oxoethyl)-3-(methanesulfonyl hydrazino carbonyl) pyridinium bromide,

(aj) 1-(2-thien-2'-yl-2-oxoethyl)-3-(ethoxycarbonyl hydrazino carbonyl) pyridinium bromide,

(ak) 1-(2-thien-2'-yl-2-oxoethyl)-3-(isopropylsulfonyl hydrazino carbonyl) pyridinium bromide,

(al) 1-(2-thien-2'-yl-2-oxoethyl)-3-(methanesulfonyl hydrazino carbonyl)-5-bromo pyridinium bromide,

(am) 1-(2-(2-ethoxy carbonyl pyrrolidin-1-yl)-2-oxoethyl)-3-(methanesulfonyl hydrazino carbonyl) pyridinium chloride,

(an) 1-(2-(5-methyl-thien-2-yl)-2-oxoethyl)-3-(methanesulfonyl hydrazino carbonyl) pyridinium chloride,

(ao) 1-(2-(4-carbethoxy-thiazolidin-3-yl)-2-oxoethyl)-3-(methanesulfonyl hydrazino carbonyl) pyridinium chloride,

(ap) 1-(2-(4-benzyl piperidin-1-yl)-2-oxoethyl)-3-(methanesulfonyl hydrazino carbonyl) pyridinium chloride,

(aq) N,N'-bis[3-carbonyl-1-(2-(2-ethoxycarbonyl pyrrolidin-1-yl)-2-oxoethyl) pyridinium] hydrazine dichloride,

(ar) 1-(2-phenylamino-2-oxoethyl)-4-[2-(benzoyloxy) ethylamino carbonyl] pyridinium chloride,

(as) 1-(2-thien-2'-yl-2-oxoethyl)-3-(phenyl hydrazino carbonyl) pyridinium bromide,

(at) 1-(2-thien-2'-yl-2-oxoethyl)-3-(p-methoxy phenyl sulfonyl hydrazino carbonyl) pyridinium bromide,

(au) 1-(2-ethoxy-2-oxoethyl)-3-(phenylaminocarbonyl hydrazino carbonyl) pyridinium bromide,

(av) 1-(2-ethoxy-2-oxoethyl)-3-(p-toluene sulfonyl hydrazino carbonyl) pyridinium bromide,

(aw) 1-(2-phenyl-2-oxoethyl)-3-(phenylamino carbonyl hydrazino carbonyl) pyridinium bromide,

(ax) 1-(2-phenylamino-2-oxoethyl)-3-(benzyl sulfonyl hydrazino carbonyl) pyridinium chloride,

(ay) 1-(2-phenyl-2-oxoethyl)-4-(methanesulfonyl hydrazino carbonyl) pyridinium bromide,

(az) 1-(2-phenyl-2-oxoethyl)-3-(phenyl hydrazino carbonyl) pyridinium bromide, -

(ba) 1-(2-ethoxy-2-oxoethyl)-4-[2-(benzoyloxy)ethylamino carbonyl] pyridinium bromide,

(bb) 1-(2-ethoxy-2-oxoethyl)-3-(phenyl hydrazino carbonyl) pyridinium bromide,

(be) 1-(2-phenyl-2-oxoethyl)-3-(p-methoxyphenyl sulfonyl hydrazino carbonyl) pyridinium bromide,

(bd) 1-(2-phenyl-2-oxoethyl)- 4-[2-(benzoyloxy) ethyl amino carbonyl] pyridinium bromide and

(be) 1-(2-ethoxy-2-oxoethyl)- 4-(p-methanesulfonyl hydrazino carbonyl) pyridinium bromide.

**Patentansprüche**

**1.** Ein Pyridiniumderivat, das aus der aus den folgenden Verbindungen bestehenden Gruppe gewählt ist:

(a) N,N'-Bis[3-carbonyl-1-(2-thien-2'-yl-2-oxoethyl)-pyridinium]hydrazindichlorid,

(b) N,N'-Bis[3-carbonyl-1-(2-cyclopropylamino-2-oxoethyl)pyridinium]hydrazindichlorid,

(c) 1-(2-Phenylamino-2-oxoethyl)-4-(phenylsulfonylhydrazinocarbonyl)pyridiniumchlorid und pharmazeutisch akzeptable Salze davon,

(d) 1-(2-(2',4-Dichlorophenyl)-2-oxoethyl)-3-(2(methoxy)ethyloxycarbonyl)pyridiniumbromid und pharmazeutisch akzeptable Salze davon,

(e) 1-(2-Phenylamino-2-oxoethyl)-3-((benzoyloxy)ethylaminocarbonyl)pyridiniumchlorid und pharmazeutisch akzeptable Salze davon,

(f) 1-(2-Thien-2'-yl-2-oxoethyl)-3-(phenylaminocarbonylhydrazinocarbonyl)pyridiniumbromid und pharmazeutisch akzeptable Salze davon;

(g) 1-(2-Phenyl-2-oxoethyl)-3-(2-(acetoxy)ethylaminocarbonyl)pyridiniumbromid und pharmazeutisch akzeptable Salze davon,

(h) 1-(2-Phenylamino-2-oxoethyl)-3-(phenylsulfonylhydrazinocarbonyl)pyridiniumchlorid und pharmazeutisch akzeptable Salze davon,

(i) 1-(2-Phenylamino-2-oxoethyl)-3-((4-methylphenyl)-sulfonylhydrazinocarbonyl)pyridiniumchlorid und pharmazeutisch akzeptable Salze davon,

(j) 1-(2-Phenyl-2-oxoethyl)-3-(2-(benzoyloxy)ethyloxycarbonyl)pyridiniumbromid und pharmazeutisch akzeptable Salze davon,

(k) 1-(2-Thien-2'-yl-2-oxoethyl)-3-(phenylcarbonylhydrazinocarbonyl)pyridiniumbromid und pharmazeutisch akzeptable Salze davon,

(l) 1-(2-Ethoxy-2-oxoethyl)-3-((phenylmethyl)sulfonylhydrazinocarbonyl)pyridiniumbromid und pharmazeutisch akzeptable Salze davon,

(m) 1-(2-Phenyl-2-oxoethyl)-3-((phenylmethyl)sulfonylhydrazinocarbonyl)pyridiniumbromid und pharmazeutisch akzeptable Salze davon,

(n) N,N'-Bis[3-carbonyl-1-(2-furan-2'-yl-2-oxoethyl)-pyridinium] hydrazindibromid,

(o) 1-(2',4'-Dichlorophenyl-2-oxoethyl)-3-(2-methoxyethylaminocarbonyl)pyridiniumbromid,

(p) 1-(2-Thien-2'-yl-2-oxoethyl)-3-((2-methoxyethyl)-aminocarbonyl)-5-bromopyridiniumchlorid,

(q) 1-(2-Thien-2'-yl-2-oxoethyl)-3-(methansulfonylhydrazinocarbonyl)pyridiniumchlorid,

(r) 1-(2-Thien-2'-yl-2-oxoethyl)-3-(2-(2-chloro-3-pyridoylhydrazinocarbonyl)pyridiniumchlorid,

(s) 1-(2-Cyclopropylamino-2-oxoethyl)-3-(2-methoxyethylaminocarbonyl)pyridiniumchlorid,

(t) 1-(2-Isopropylamino-2-oxoethyl)-3-(2-methylsulfonylhydrazinocarbonyl)pyridiniumchlorid,

(u) 1-(2-Phenylamino-2-oxoethyl)-3-((2-(1-oxo-3-cyclohexyl)-propyl)hydrazinocarbonyl)pyridiniumbromid,

(v) 1-(2-Thien-2'-yl-2-oxoethyl)-3-[2-(benzoyloxy)-ethylaminocarbonyl]pyridiniumbromid,

(w) 1-(4-Ethoxy-2,4-dioxobutyl)-3-(2-(benzoxyloxy)-ethylaminocarbonyl)pyridiniumchlorid,

(x) 1-(2-Thien-2'-yl-2-oxoethyl)-3-[1-oxo-1-(2-methoxycarbonyl)pyridyl]hydrazinopyridiniumchlorid,

(y) 1-[1-(2-Thien-2'-yl-2-oxoethyl)-5-aminocarbonyl-3-carbonylpyridinium]-2-[1-(2-thien-2'-yl-2-oxoethyl)-3-carbonylpyridinium]hydrazindichlorid,

(z) 1-(2-Thien-2'-yl-2-oxoethyl)-3-(trifluoromethansulfonylhydrazinocarbonyl)pyridiniumbromid,

(aa) 1-[1-(2-Thien-2'-yl-2-oxoethyl)-6-methyl-3-carbonylpyridinium]-2-[1-(2-thien-2'-yl-2-oxoethyl)-3-carbonylpyridinium]hydrazindichlorid,

(ab) N,N'-Bis[3-carbonyl-1-(2-(5-methyl-thien-2-yl)-2-oxoethyl)pyridinium]hydrazindichlorid,

(ac) N,N'-Bis[3-carbonyl-1-(2-(5-chloro-thien-2-yl)-2-oxoethyl)pyridinium]hydrazindichlorid,

(ad) 1-(2-Thien-2'-yl-2-oxoethyl)-3-(methansulfonylhydrazinocarbonyl)-6-methylpyridiniumbromid,

(ae) N,N'-Bis[3-carbanyl-1-(2-(4-nitro-thien-2-yl)-2-oxoethyl)pyridinium]hydrazindichlorid,

(af) 1-(2-Phenylamino-2-oxoethyl)-3-(phenylhydrazinocarbonyl)pyridiniumchlorid,

(ag) 1-(2-(4-Nitro-thien-2-yl)-2-oxoethyl)-3-(methansulfonylhydrazinocarbonyl)pyridiniumbromid,

(ah) 1-(2-(5-Nitro-thien-2-yl)-2-oxoethyl)-3-(methansulfonylhydrazinocarbonyl)pyridiniumchlorid,

(ai) 1-(2-(5-Chloro-thien-2-yl)-2-oxoethyl)-3-(methansulfonylhydrazinocarbonyl)pyridiniumbromid,

(aj) 1-(2-Thien-2'-yl-2-oxoethyl)-3-(ethoxycarbonylhydrazinocarbonyl)pyridiniumbromid,

(ak) 1-(2-Thien-2'-yl-2-oxoethyl)-3-(isopropylsulfonylhydrazinocarbonyl)pyridiniumbromid,

(al) 1-(2-Thien-2'-yl-2-oxoethyl)-3-(methansulfonylhydrazinocarbonyl)-5-bromopyridiniumbromid,

(am) 1-(2-(2-Ethoxycarbonylpyrrolidin-1-yl)-2-oxoethyl)-3-(methansulfonylhydrazinocarbonyl)pyridiniumchlorid,

(an) 1-(2-(5-Methyl-thien-2-yl)-2-oxoethyl)-3-(methansulfonylhydrazinocarbonyl)pyridiniumchlorid,

(ao) 1-(2-(4-Carbethoxythiazolidin-3-yl)-2-oxoethyl)-3-(methansulfonylhydrazinocarbonyl)pyridiniumchlorid,

(ap) 1-(2-(4-Benzylpiparidin-1-yl)-2-oxoethyl)-3-(methansulfonylhydrazinocarbonyl)pyridiniumchlorid,

(aq) N,N'-Bis[3-carbonyl-2-(2-(2-ethoxycarbonylpyrrolidin-1-yl)-oxoethyl)pyridinium]hydrazindichlorid,

(ar) 1-(2-Phenylamino-2-oxoethyl)-4-[2-(benzoyloxy)-ethylaminocarbonyl]pyridiniumchlorid,

(as) 1-(2-Thien-2'-yl-2-oxoethyl)-3-(phenylhydrazinocarbonyl)pyridiniumbromid,

(at) 1-(2-Thien-2'-yl-2-oxoethyl)-3-(p-methoxyphenylsulfonylhydrazinocarbonyl)pyridiniumbromid,

(au) 1-(2-Ethoxy-2-oxoethyl)-3-(phenylaminocarbonylhydrazinocarbonyl)pyridiniumbromid,

(av) 1-(2-Ethoxy-2-oxoethyl)-3-(p-toluolsulfonylhydrazinocarbonyl) pyridiniumbromid,

(aw) 1-(2-Phenyl-2-oxoethyl)-3-(phenylaminocarbonylhydrazinocarbonyl)pyridiniumbromid,

(ax) 1-(2-Phenylamino-2-oxoethyl)-3-benzylsulfonylhydrazinocarbonyl)pyridiniumchlorid,

(ay) 1-(2-Phenyl-2-oxoethyl)-4-(methansulfonylhydrazinocarbonyl)pyridiniumbromid,

(az) 1-(2-Phenyl-2-oxoethyl)-3-(phenylhydrazinocarbonyl)pyridiniumbromid,

(ba) 1-(2-Ethoxy-2-oxoethyl)-4-[2-(benzoyloxy)ethylaminocarbonyl]pyridiniumbromid,

(bb) 1-(2-Ethoxy-2-oxoethyl)-3-(phenylhydrazinocarbonyl)pyridiniumbromid,

(be) 1-(2-Phenyl-2-oxoethyl)-3-(p-methoxyphenylsulfonylhydrazinocarbonyl)pyridiniumbromid,

(bd) 1-(2-Phenyl-2-oxoethyl)-4-[2-(benzoyloxy)ethylaminocarbonyl]pyridiniumbromid und

(be) 1-(2-Ethoxy-2-oxoethyl)-4-(p-methansulfonylhydrazinocarbonyl)pyridiniumbromid.

**2.** Ein Prozess zur Herstellung von Verbindungen, wie in Anspruch 1 beansprucht, der folgendes umfasst:

i) Rücklaufenlassen eines Pyridinderivats, gewählt aus:

(a) N,N'-Bis(nicotinyl)hydrazin
(b) 3-[(2-Pyridyl)hydrazinocarbonyl]pyridin
(c) 3-[(2-Methansulfonyl)hydrazinocarbonyl]pyridin
(d) 3-[(2-Benzoyloxy)ethylaminocarbonyl]pyridin
(e) 3- [(2-Phenylsulfonyl)hydrazinocarbonyl]pyridin
(f) 3-[(2-Acetoxy)ethyloxycarbonyl]pyridin
(g) 3- [(2-Benzoyloxy)ethoxycarbonyl]pyridin
(h) 3-[(2-Methoxy)ethyloxycarbonyl]pyridin
(i) 3-[(2-Phenylaminocarbonyl)hydrazinocarbonyl]pyridin
(j) 3-[(2-Acetoxy)ethylaminocarbonyl]pyridin
(k) 3-[(2-(4-Methylphenylsulfonyl)hydrazinocarbonyl]-pyridin
(l) 3-[(2-Benzoyl)-hydrazinocarbonyl]pyridin
(m) 3-[(2-Phenylmethansulfonyl)hydrazinocarbonyl]-pyridin
(n) 3-[(2-(3-Cyclohexylpropanoyl)hydrazinocarbonyl]-pyridin
(o) 3-[(2-Methoxy)ethylaminocarbonyl]pyridin
(p) 3-[(1-Oxo-1-(2-methoxycarbonyl)pyridyl]hydrazinopyridin

mit einem quaternierenden Mittel, gewählt aus der Gruppe, bestehend aus:

(a) 2-Bromoacetylthiophen
(b) 2-Chloroacetylthiophen
(c) Phenacylbromid
(d) Phenacylchlorid
(e) 2,4-Dichlorophenacylbromid
(f) N-Phenylchloroacetamid
(g) N-Cyclopropylchloroacetamid
(h) Ethylbromoacetat
(i) Bromoacetylfuran
(j) N-Isopropylchloroacetamid
(k) X-Chloroacetyl-2-pyrrolidinon und
(l) Chloressigsäure

in einem alkoholischen Lösungsmittel, gewählt aus der Gruppe, bestehend aus:

(a) Methanol
(b) Ethanol
(d) N-Propanol und
(e) Isopropanol

und/oder einem schwersiedenden Lösungsmittel, gewählt aus der Gruppe, bestehend aus:

(a) Toluol
(b) o-Xylol
(c) m-Xylol
(d) p-Xylol und
(e) Xylolen

während 6-48 Stunden.
(ii) Isolieren der Verbindung von Schritt (i).

**3.** Die Verwendung der Verbindung, wie in Anspruch 1 beansprucht, bei der Herstellung eines Medikaments für diabetische Komplikationen und altersbedingte Krankheiten, einschließlich Nierenkrankheit, Nervenschaden, Retinopathie, Atherosklerose, Mikroangiopathie, endothelialer Funktionsstörungen, dermatologischer Zustände, Verfärbung von Zähnen und anderer organischer Funktionsstörungen.

**4.** Eine pharmazeutische Zusammensetzung zur Behandlung diabetischer Komplikationen und altersbedingter Krankheiten, die eine pharmazeutisch wirksame Menge einer oder mehrerer Verbindungen, wie in Anspruch 1 beansprucht, oder ein pharmazeutisch akzeptables Salz bzw. Salze davon in Beimischung mit einem pharmazeutisch akzeptablen Träger, Verdünner, Lösungsmittel oder Arzneistoffträger umfasst.

**5.** Die pharmazeutische Zusammensetzung, wie in Anspruch 4 beansprucht, in Form einer oralen Rezeptur.

**6.** Die pharmazeutische Zusammensetzung, wie in Anspruch 5 beansprucht, wobei besagter pharmazeutisch akzeptabler Träger aus der aus Stärke, Laktose, Polyvinylpyrolidon (K-30), Talk und Magnesiumstearat bestehenden Gruppe gewählt ist.

**7.** Die pharmazeutische Zusammensetzung, wie in Anspruch 4 beansprucht, in Form einer parenteralen Rezeptur.

**8.** Ein Verfahren zur Herstellung einer parenteralen Rezeptur, wie in Anspruch 7 beansprucht, das das Auflösen einer oder mehr Verbindungen, wie in Anspruch 1 definiert, in Polyethylenglykol 400 und Verdünnen der so erhaltenen Lösung mit einer isotonischen Lösung oder Wasser auf eine gewünschte Konzentration umfasst.

**9.** Pharmazeutische Zusammensetzung, wie in Anspruch 6 beansprucht, in Form einer Lotion, Mundspülung und Zahnpasta.

**10.** Verwendung der Verbindung von Anspruch 1 zur Fertigung eines Medikaments zur Behandlung eines Diabetespatienten durch Brechen eines vorgeformten AGE innerhalb besagten Patienten, welche das Verabreichen einer effizienten Menge besagter Verbindung entweder allein oder in Kombination mit anderen Drogen zur Antidiabetestherapie umfasst.

**11.** Verwendung der Verbindung von Anspruch 1 zur Fertigung eines Medikaments zur Vorbeugung oder Behandlung von durch Diabetes und altersbedingten Komplikationen verursachten Krankheiten, welche das Verabreichen einer effizienten Menge besagter Verbindung, entweder allein oder in Kombination mit einem pharmazeutisch akzeptablen Träger, Verdünner oder Arzneimittelträger, an einen Patienten, der dessen bedarf, umfasst.

**12.** Die Verwendung, wie in Anspruch 11 beansprucht, wobei die vorgebeugte oder behandelte Krankheit eine nephrologische Störung, neurologische Störung, Atherosklerose, Retina-Störung, dermatologische Störung, nicht-enzymbedingtes Braunwerden der Mundhöhle, Endothel- oder andere Organfunktionsstörung und Wachstumsstörung ist.

**13.** Die Verwendung gemäß Anspruch 12, wobei besagte Verbindung aus der Gruppe gewählt ist, bestehend aus:

(a) N,N'-Bis[3-carbonyl-1-(2-thien-2'-yl-2-oxoethyl)-pyridinium]hydrazindichlorid,

(b) N,N'-Bis[3-carbonyl-1-(2-cyclopropylamino-2-oxoethyl) pyridinium]hydrazindichlorid,

(c) 1-(2-Phenylamino-2-oxoethyl)-4-(phenylsulfonylhydrazinocarbonyl)pyridiniumchlorid und pharmazeutisch akzeptable Salze davon,

(d) 1-(2-(2',4-Dichlorophenyl)-2-oxoethyl)-3-(2(methoxy)ethyloxycarbonyl)pyridiniumbromid und pharmazeutisch akzeptable Salze davon,

(e) 1-(2-Phenylamino-2-oxoethyl)-3-((benzoyloxy)ethylaminocarbonyl)pyridiniumchlorid und pharmazeutisch akzeptable Salze davon,

(f) 1-(2-Thien-2'-yl-2-oxoethyl)-3-(phenylaminocarbonylhydrazinocarbonyl)pyridiniumbromid und pharmazeutisch akzeptable Salze davon;

(g) 1-(2-Phenyl-2-oxoethyl)-3-(2-(acetoxy)ethylaminocarbonyl)pyridiniumbromid und pharmazeutisch akzeptable Salze davon,

(h) 1-(2-Phenylamino-2-oxoethyl)-3-(phenylsulfonylhydrazinocarbonyl)pyridiniumchlorid und pharmazeutisch akzeptable Salze davon,

(i) 1-(2-Phenylamino-2-oxoethyl)-3-((4-methyl-phenyl)sulfonylhydrazinocarbonyl)pyridiniumchlorid und pharmazeutisch akzeptable Salze davon,

(j) 1-(2-Phenyl-2-oxoethyl)-3-(2-(benzoyloxy)ethyloxycarbonyl)pyridiniumbramid und pharmazeutisch akzeptable Salze davon,

(k) 1-(2-Thien-2'-yl-2-oxoethyl)-3-(phenylcarbonylhydrazinocarbonyl)pyridiniumbromid und pharmazeutisch akzeptable Salze davon,

(l) 1-(2-Ethoxy-2-oxoethyl)-3-((phenylmethyl)sulfonylhydrazinocarbonyl)pyridiniumbromid und pharmazeutisch akzeptable Salze davon,

(m) 1-(2-Phenyl-2-oxoethyl)-3-((phenylmethyl)sulfonylhydrazinocarbonyl)pyridiniumbromid und pharmazeutisch akzeptable Salze davon,

(n) N,N'-Bis[3-carbonyl-1-(2-furan-2'-yl-2-oxoethyl)-pyridinium] hydrazindibromid,

(o) 1-(2',4'-Dichlorophenyl-2-oxoethyl)-3-(2-methoxyethylaminocarbonyl)pyridiniumbromid,

(p) 1-(2-Thien-2'-yl-2-oxoethyl)-3-((2-methoxyethyl)-aminocarbonyl)-5-bromopyridiniumchlorid,

(q) 1-(2-Thien-2'-yl-2-oxoethyl)-3-(methansulfonylhydrazinocarbonyl)pyridiniumchlorid,

(r) 1-(2-Thien-2'-yl-2-oxoethyl)-3-(2-(2-chloro-3-pyridoylhydrazinocarbonyl)-pyridiniumchlorid,

(s) 1-(2-Cyclopropylamino-2-oxoethyl)-3-(2-methoxyethylaminocarbonyl)pyridiniumchlorid,

(t) 1-(2-Isopropylamino-2-oxoethyl)-3-(2-methylsulfonylhydrazinocarbonyl)-pyridiniumchlorid,

(u) 1-(2-Phenylamino-2-oxoethyl)-3-({2-(1-oxo-3-cyclohexyl)propyl}hydrazinocarbonyl)-pyridiniumbromid,

(v) 1-(2-Thien-2'-yl-2-oxoethyl)-3-[2-(benzoyloxy)-ethylaminocarbonyl]pyridiniumbromid,

(w) 1-(4-Ethoxy-2,4-dioxobutyl)-3-(2-(benzoxyloxy) - ethylaminocarbonyl)-pyridiniumchlorid,

(x) 1-(2-Thien-2'-yl-2-oxoethyl)-3-[1-oxo-1-(2-methoxycarbonyl)pyridyl]hydrazinopyridiniumchlorid,

(y) 1-[1-(2-Thien-2'-yl-2-oxoethyl)-5-aminocarbonyl-3-carbonylpyridinium]-2-[1-(2-thien-2'-yl-2-oxoethyl)-3-carbonylpyridinium]hydrazinodichlorid,

(z) 1-(2-Thien-2'-yl-2-oxoethyl)-3-(trifluoromethansulfonylhydrazinocarbonyl)pyridiniumbromid,

(aa) 1-[1-(2-Thien-2'-yl-2-oxoethyl)-6-methyl-3-carbonylpyridinium]-2-[1-(2-thien-2'-yl-2-oxoethyl)-3-carbonylpyridinium]hydrazindichlorid,

(ab) N,N'-Bis[3-carbonyl-1-(2-(5-methyl-thien-2-yl)-2-oxoethyl)pyridinium]hydrazindichlorid,

(ac) N,N'-Bis[3-carbonyl-1-(2-(5-chlor-thien-2-yl)-2-oxoethyl)pyridinium]hydrazindichlorid,

(ad) 1-(2-Thien-2'-yl-2-oxoethyl)-3-(methansulfonylhydrazinocarbonyl)-6-methylpyridiniumbromid,

(ae) N,N'-Bis[3-carbonyl-1-(2-(4-nitro-thien-2-yl)-2-oxoethyl)pyridinium]hydrazindichlorid,

(af) 1-(2-Phenylamino-2-oxoethyl)-3-(phenylhydrazinocarbonyl)pyridiniumchlorid,

(ag) 1-(2-(4-Nitro-thien-2-yl)-2-oxoethyl)-3-(methansulfonylhydrazinocarbonyl)pyridiniumbromid,

(ah) 1-(2-(5-Nitro-thien-2-yl)-2-oxoethyl)-3-(methansulfonylhydrazinocarbonyl)pyridiniumchlorid,

(ai) 1-(2-(5-Chloro-thien-2-yl)-2-oxoethyl)-3-(methansulfonylhydrazinocarbonyl)pyridiniumbromid,

(aj) 1-(2-Thien-2'-yl-2-oxoethyl)-3-(ethoxycarbonylhydrazinocarbonyl)pyridiniumbromid,

(ak) 1-(2-Thien-2'-yl-2-oxoethyl)-3-(isopropylsulfonylhydrazinocarbonyl)pyridiniumbromid,

(al) 1-(2-Thien-2'-yl-2-oxoethyl)-3-(methansulfonylhydrazinocarbonyl)-5-bromopyridiniumbromid,

(am) 1-(2-(2-Ethoxycarbonylpyrrolidin-1-yl)-2-oxoethyl)-3-(methansulfonylhydrazinocarbonyl)pyridiniumchlorid,

(an) 1-(2-(5-Methyl-thien-2-yl)-2-oxoethyl)-3-(methansulfonylhydrazinocarbonyl)pyridiniumchlorid,

(ao) 1-(2-(4-Carbethoxythiazolidin-3-yl)-2-oxoethyl)-3-(methansulfonylhydrazinocarbonyl)pyridiniumchlorid,

(ap) 1-(2-(4-Benzylpiperidin-1-yl)-2-oxoethyl)-3-(methansulfonylhydrazinocarbonyl)pyridiniumchlorid,

(aq) N,N'-Bis[3-carbonyl-2-(2-(2-ethoxycarbonylpyrrolidin-1-yl)-oxoethyl)pyridinium]hydrazindichlorid,

(ar) 1-(2-Phenylamino-2-oxoethyl)-4-[2-(benzoyloxy)-ethylaminocarbonyl]pyridiniumchlorid,

(as) 1-(2-Thien-2'-yl-2-oxoethyl)-3-(phenylhydrazinocarbonyl)pyridiniumbromid,

(at) 1-(2-Thien-2'-yl-2-oxoethyl)-3-(p-methoxyphenylsulfonylhydrazinocarbonyl)pyridiniumbromid,

(au) 1-(2-Ethoxy-2-oxoethyl)-3-(phenylaminocarbonylhydrazinocarbonyl)pyridiniumbromid,

(av) 1-(2-Ethoxy-2-oxoethyl)-3-(p-toluolsulfonylhydrazinocarbonyl)pyridiniumbromid,

(aw) 1-(2-Phenyl-2-oxoethyl)-3-(phenylaminocarbonylhydrazinocarbonyl)pyridiniumbromid,

(ax) 1-(2-Phenylamino-2-oxoethyl)-3-benzylsulfonylhydrazinocarbonyl)pyridiniumchlorid,

(ay) 1-(2-Phenyl-2-oxoethyl)-4-(methansulfonylhydrazinocarbonyl)pyridiniumbromid,

(az) 1-(2-Phenyl-2-oxoethyl)-3-(phenylhydrazinocarbonyl)pyridiniumbromid,

(ba) 1-(2-Ethoxy-2-oxoethyl)-4-[2-(benzoyloxy)ethylaminocarbonyl]pyridiniumbromid,

(bb) 1-(2-Ethoxy-2-oxoethyl)-3-(phenylhydrazinocarbonyl)pyridiniumbromid,

(be) 1-(2-Phenyl-2-oxoethyl)-3-(p-methoxyphenylsulfonylhydrazinocarbonyl)pyridiniumbromid,

(bd) 1-(2-Phenyl-2-oxoethyl)-4-[2-(benzoyloxy)ethylaminocarbonyl]pyridiniumbromid und

(be) 1-(2-Ethoxy-2-oxoethyl)-4-(p-methansulfonylhydrazinocarbonyl)pyridiniumbromid.

## Revendications

**1.** Dérivé de pyridiniumium choisi parmi le groupe constitué par les composés suivants :

(a) le dichlorure de la N,N'-bis[3-carbonyl-1-(2-thién-2'-yl-2-oxoéthyl)pyridinium]hydrazine,

(b) le dichlorure de la N,N'-bis[3-carbonyl-1-(2-cyclopropylamino-2-oxo-éthyl)pyridinium]hydrazine,

(c) le chlorure du 1-(2-phénylamino-2-oxoéthyl)-4-(phénylsulfonylhydrazinocarbonyl)pyridinium et ses sels pharmaceutiquement acceptables,

(d) le bromure du 1-(2-(2',4'-dichlorophényl)-2-oxoéthyl)-3-(2(méthoxy)éthyloxycarbonyl)pyridinium et ses sels pharmaceutiquement acceptables,

(e) le chlorure du 1-(2-pénylamino-2-oxoéthyl)-3-((benzoyloxy)éthylaminocarbonyl)pyridinium et ses sels pharmaceutiquement acceptables,

(f) le bromure du 1-(2-thién-2'-yl-2-oxoéthyl)-3-(phénylaminocarbonylhydrazinocarbonyl)pyridinium et ses sels pharmaceutiquement acceptables;

(g) le bromure du 1-(2-phényl-2-oxoéthyl)-3-(2-(acétoxy)éthylaminocarbonyl)pyridinium et ses sels pharmaceutiquement acceptables,

(h) le chlorure du 1-(2-phénylamino-2-oxoéthyl)-3-(phénylsulfonylhydrazinocarbonyl)pyridinium et ses sels pharmaceutiquement acceptables,

(i) le chlorure du 1-(2-phénylamino-2-oxoéthyl)-3-((4-méthylphényl)sulfonylhydrazinocarbonyl)pyridinium et ses sels pharmaceutiquement acceptables,

(j) le bromure du 1-(2-phényl-2-oxoéthyl)-3-(2-(benzoyloxy)éthyloxycarbonyl)pyridinium et ses sels pharmaceutiquement acceptables,

(k) le bromure du 1-(2-thién-2'-yl-2-oxoéthyl)-3-(phénylcarbonylhydrazinocarbonyl)pyridinium et ses sels pharmaceutiquement acceptables,

(l) le bromure du 1-(2-éthoxy-2-oxoéthyl)-3-((phénylméthyl)sulfonylhydrazinocarbonyl)pyridinium et ses sels pharmaceutiquement acceptables,

(m) le bromure du 1-(2-phényl-2-oxoéthyl)-3-((phénylméthyl)sulfonylhydrazinocarbonyl)pyridinium et ses sels pharmaceutiquement acceptables,

(n) le dibromure de la N,N'-bis[3-carbonyl-1-(2-furan-2'-yl-2-oxoéthyl)pyridinium]hydrazine,

(o) le bromure du 1-(2',4'-dichlorophényl-2-oxoéthyl)-3-(2-méthoxyéthylaminocarbonyl)pyridinium,

(p) le chlorure du 1-(2-thién-2'-yl-2-oxoéthyl)-3-((2-méthoxyéthyl)amincarbonyl)-5-bromopyridinium,

(q) le chlorure du 1-(2-thién-2'-yl-2-oxoéthyl)-3-(méthansulfonylhydrazinocarbonyl)pyridinium,

(r) le chlorure du 1-(2-thién-2'-yl-2-oxoéthyl)-3-(2-(2-chloro-3-pyridoylhydrazinocarbonyl)pyridinium,

(s) le chlorure du 1-(2-cyclopropylamino-2-oxoéthyl)-3-(2-méthoxyéthylaminocarbonyl)pyridinium,

(t) le chlorure du 1-(2-isopropylamino-2-oxoéthyl)-3-(2-méthylsulfonylhydrazinocarbonyl)pyridinium,

(u) le bromure du 1-(2-phénylamino-2-oxoéthyl)-3-((2-(1-oxo-3-cyclohexyl)propyl)hydrazinocarbonyl)-pyridinium,

(v) le bromure du 1-(2-thién-2'-yl-2-oxoéthyl)-3-[2-(benzoyloxy)éthylaminocarbonyl]pyridinium,

(w) le chlorure du 1-(4-éthoxy-2,4-dioxobutyl)-3-(2-(benzoxyloxy)-éthylaminocarbonyl)pyridinium,

(x) le chlorure du 1-(2-thién-2'-yl-2-oxoéthyl)-3-[1-oxo-1-(2-méthoxycarbonyl)pyridyl]hydrazinopyridinium,

(y) le dichlorure de la 1-[1-(2-thién-2'-yl-2-oxoéthyl)-5-aminocarbonyl-3-carbonylpyridinium]-2-[1-(2-thién-2'-yl-2-oxoéthyl)-3-carbonylpyridinium]hydrazine,

(z) le bromure du 1-(2-thién-2'-yl-2-oxoéthyl)-3-(trifluorométhanesulfonylhydrazinocarbonyl)pyridinium,

(aa) le dichlorure de la 1-[1-(2-thién-2'-yl-2-oxoéthyl)-6-méthyl-3-carbonylpyridinium]-2-[1-(2-thién-2'-yl-2-oxoéthyl)-3-carbonylpyridinium]hydrazine,

(ab) le dichlorure de la N,N'-bis[3-carbonyl-1-(2-(5-méthylthién-2-yl)-2-oxoéthyl)pyridinium]hydrazine,

(ac) le dichlorure de la N,N'-bis[3-carbonyl-1-(2-(5-chlorothién-2-yl)-2-oxoéthyl)pyridinium]hydrazine,

(ad) le bromure du 1-(2-thién-2'-yl-2-oxoéthyl)-3-(méthanesulfonylhydrazinocarbonyl)-6-méthylpyridinium,

(ae) le dichlorure de la N,N'-bis[3-carbonyl-1-(2-(4-nitrothién-2-yl)-2-oxoéthyl)pyridinium]hydrazine,

(af) le chlorure du 1-(2-phénylamin-2-oxoéthyl)-3-(phénylhydrazinocarbonyl)pyridinium,

(ag) le bromure du 1-(2-(4-nitrothién-2-yl)-2-oxoéthyl)-3-(méthanesulfonylhydrazinocarbonyl)pyridinium,

(ah) le chlorure du 1-(2-(5-nitrothién-2-yl)-2-oxoéthyl)-3-(méthanesulfonylhydrazinocarbonyl)-pyridinium,

(ai) le bromure du 1-(2-(5-chlorothién-2-yl)-2-oxoéthyl)-3-(méthanesulfonylhydrazinocarbonyl)-pyridinium,

(aj) le bromure du 1-(2-thién-2'-yl-2-oxoéthyl)-3-(éthoxycarbonylhydrazinocarbonyl)pyridinium,

(ak) le bromure du 1-(2-thién-2'-yl-2-oxoéthyl)-3-(isopropylsulfonylhydrazinocarbonyl)pyridinium,

(al) le bromure du 1-(2-thién-2'-yl-2-oxoéthyl)-3-(méthanesulfonylhydrazinocarbonyl)-5-bromopyridinium,

(am) le chlorure du 1-(2-(2-éthoxycarbonylpyrrolidin-1-yl)-2-oxoéthyl)-3-(méthanesulfonylhydrazinocarbonyl)-pyridinium,

(an) le chlorure du 1-(2-(5-méthylthién-2-yl)-2-oxoéthyl)-3-(méthanesulfonylhydrazinocarbonyl)-pyridinium,

(ao) le chlorure du 1-(2-(4-carbéthoxythiazolidin-3-yl)-2-oxoéthyl)-3-(méthanesulfonylhydrazinocarbonyl)-pyridinium,

(ap) le chlorure du 1-(2-(4-benzylpipéridin-1-yl)-2-oxoéthyl)-3-(méthanesulfonylhydrazinocarbonyl)-pyridinium,

(aq) le dichlorure de la N,N'-bis[3-carbonyl-2-(2-(2-éthoxycarbonylpyrrolidin-1-yl)-oxoéthyl)pyridinium]-hydrazine,

(ar) le chlorure du 1-(2-phénylamino-2-oxoéthyl)-4-[2-(benzoyloxy)éthylaminocarbonyl]pyridinium,

(as) le bromure du 1-(2-thién-2'-yl-2-oxoéthyl)-3-(phénylhydrazinocarbonyl)pyridinium,

(at) le bromure du 1-(2-thién-2'-yl-2-oxoéthyl)-3-(p-méthoxyphénylsulfonylhydrazinocarbonyl)pyridinium,

(au) le bromure du 1-(2-éthoxy-2-oxoéthyl)-3-(phénylaminocarbonylhydrazinocarbonyl)pyridinium,

(av) le bromure du 1-(2-éthoxy-2-oxoéthyl)-3-(p-toluènesulfonylhydrazinocarbonyl)pyridinium,

(aw) le bromure du 1-(2-phényl-2-oxoéthyl)-3-(phénylaminocarbonylhydrazinocarbonyl)pyridinium,

(ax) le chlorure du 1-(2-phénylamino-2-oxoéthyl)-3-benzylsulfonylhydrazinocarbonyl)pyridinium,

(ay) le bromure du 1-(2-phényl-2-oxoéthyl)-4-(méthanesulfonylhydrazinocarbonyl)pyridinium,

(az) le bromure du 1-(2-phényl-2-oxoéthyl)-3-(phénylhydrazinocarbonyl)pyridinium,

(ba) le bromure du 1-(2-éthoxy-2-oxoéthyl)-4-[2-(benzoyloxy)éthylaminocarbonyl]pyridinium,

(bb) le bromure du 1-(2-éthoxy-2-oxoéthyl)-3-(phénylhydrazinocarbonyl)pyridinium,

(bc) le bromure du 1-(2-phényl-2-oxoéthyl)-3-(p-méthoxyphénylsulfonylhydrazinocarbonyl)pyridinium,

(bd) le bromure du 1-(2-phényl-2-oxoéthyl)-4-[2-(benzoyloxy)éthylaminocarbonyl]pyridinium et

(be) le bromure du 1-(2-éthoxy-2-oxoéthyl)-4-(p-méthanesulfonylhydrazinocarbonyl)pyridinium.

**2.** Procédé pour la préparation de composés selon la revendication 1, qui comprend le fait de

i) chauffer à reflux un dérivé de pyridine choisi parmi le groupe comprenant :

(a) la N,N'-bis(nicotinyl)hydrazine
(b) la 3-[(2-pyridyl)hydrazinocarbonyl]pyriridine
(c) la 3-[(2-méthanesulfonyl)hydrazinocarbonyl]-pyriridine
(d) la 3-[(2-benzoyloxy)éthylaminocarbonyl]pyriridine
(e) la 3-[(2-phénylsulfonyl)hydrazinocarbonyl]-pyriridine
(f) la 3-[(2-acétoxy)éthyloxycarbonyl]pyriridine
(g) la 3-[(2-benzoyloxy)éthoxycarbonyl]pyriridine
(h) la 3-[(2-méthoxy)éthyloxycarbonyl]pyriridine
(i) la 3-[(2-phénylaminocarbonyl)hydrazinocarbonyl]-pyriridine
(j) la 3-[(2-acétoxy)éthylaminocarbonyl]pyriridine
(k) la 3-[(2-(4-méthylphénylsulfonyl)hydrazinocarbonyl]pyriridine
(l) la 3-[(2-benzoyl)hydrazinocarbonyl]pyriridine
(m) la 3-[(2-phénylméthanesulfonyl)hydrazinocarbonyl]-pyriridine
(n) la 3-[(2-(3-cyclohexylpropanoyl)hydrazinocarbonyl]-pyriridine
(o) la 3-[(2-méthoxy)éthylaminocarbonyl]pyriridine
(p) la 3-[(1-oxo-1-(2-méthoxycarbonyl)pyridyl]-hydrazinopyriridine

avec un agent de quaternisation choisi parmi le groupe constitué par :

(a) le 2-bromoacéthylthiophène
(b) le 2-chloroacéthylthiophène
(c) le bromure de phénacyle
(d) le chlorure de phénacyle

(e) le bromure de 2,4-dichlorophénacyle
(f) le N-phénylchloroacétamide
(g) le N-cyclopropylchloroacétamide
(h) le bromoacétate d'éthyle
(i) le bromoacétylfuranne
(j) le N-isopropylchloroacétamide
(k) la N-chloroacétyl-2-pyrrolidinone et
(l) l'acide chloroacétique

dans un solvant alcoolique choisi parmi le groupe comprenant :

(a) le méthanol
(b) l'éthanol
(c) le n-propanol et
(d) l'isopropanol

et/ou dans un solvant à point d'ébullition élevé choisi parmi le groupe comprenant :

(a) le toluêne
(b) le o-xylène
(c) le m-xylène
(d) le p-xylène et
(e) les xylènes

pendant un laps de temps de 6 à 48 heures ;

(ii) isoler le composé de l'étape (i).

**3.** Utilisation d'un composé selon la revendication 1, dans la préparation d'un médicament pour des complications diabétiques et pour des maladies liées au vieillissement, y compris des maladies rénales, des lésions nerveuses, la rétinopathie, l'athérosclérose, la micro-augiopathie, des troubles endothélials, des affections dermatologiques, la pigmentation dentaire et d'autres troubles organiques.

**4.** Composition pharmaceutique pour le traitement de complications diabétiques et de maladies liées au vieillissement, qui comprend une quantité pharmaceutiquement efficace d'un ou de plusieurs composés selon la revendication 1, ou d'un de leurs sels pharmaceutiquement acceptables ou plus, en mélange avec un support, un diluant, un solvant ou un excipient pharmaceutiquement acceptable.

**5.** Composition pharmaceutique selon la revendication 4, sous la forme d'une formulation orale.

**6.** Composition pharmaceutique selon la revendication 5, dans laquelle ledit support pharmaceutiquement acceptable est choisi parmi le groupe constitué par l'amidon, le lactose, la polyvinylpyrrolidone (K-30), le talc et le stéarate de magnésium.

**7.** Composition pharmaceutique selon la revendication 4, sous la forme d'une formulation parentérale.

**8.** Procédé pour la préparation d'une formulation parentérale selon la revendication 7, qui comprend le fait de dissoudre un ou plusieurs composés selon la revendication 1, dans du polyéthylèneglycol 400 et le faits de diluer la solution ainsi obtenue avec une solution isotonique ou avec de l'eau pour obtenir une concentration désirée.

**9.** Composition pharmaceutique selon la revendication 6, sous la forme d'une lotion, d'un bain de bouche ou d'un dentifrice.

**10.** Utilisation du composé selon la revendication 1, pour la préparation d'un médicament destiné à traiter un patient diabétique par rupture d'un AGE préformé, au sein du type patient, qui comprend le fait d'administrer une quantité efficace dudit composé, soit à titre individuel, soit en combinaison avec d'autres médicaments destinés à la thérapie antidiabétique.

**11.** Utilisation du composé selon un revendication 1, pour la préparation d'un médicament pour prévenir ou traiter des maladies provoquées par le diabète et des complications liées au vieillissement, qui comprend le fait d'administrer à un patient qui en a besoin, une quantité efficace dudit composé, soit à titre individuel, soit en combinaison avec un support, un diluant ou un excipient pharmaceutiquement acceptable.

**12.** Utilisation selon la revendication 11, dans lequel la maladie soumise à une prévention ou à un traitement est un trouble néphrologique, un trouble neurologique, l'athérosclérose, un trouble rétinien, un trouble dermatologique, le brunissement non enzymatique de la cavité buccale, un trouble endothélial ou un autre trouble organique et un dysfonctionnement de la croissance.

**13.** Utilisation selon la revendication 12, dans laquelle ledit composé est choisi parmi le groupe constitué par :

(a) le dichlorure de la N,N'-bis[3-carbonyl-1-(2-thién-2'-yl-2-oxoéthyl)pyridinium]hydrazine,

(b) le dichlorure de la N,N'-bis[3-carbonyl-1-(2-cyclopropylamino-2-oxo-éthyl)pyridinium]hydrazine,

(c) le chlorure du 1-(2-phénylamino-2-oxoéthyl)-4-(phénylsulfonylhydrazinocarbonyl)pyridinium et ses sels pharmaceutiquement acceptables,

(d) le bromure du 1-(2-(2',4'-dichlorophényl)-2-oxoéthyl)-3-(2(méthoxy)éthyloxycarbonyl)pyridinium et ses sels pharmaceutiquement acceptables,

(e) le chlorure du 1-(2-pénylamino-2-oxoéthyl)-3-((benzoyloxy)éthylaminocarbonyl)pyridinium et ses sels pharmaceutiquement acceptables,

(f) le bromure du 1-(2-thién-2'-yl-2-oxoéthyl)-3-(phénylaminocarbonylhydrazinocarbonyl)pyridinium et ses sels pharmaceutiquement acceptables;

(g) le bromure du 1-(2-phényl-2-oxoéthyl)-3-(2-(acétoxy)éthylaminocarbonyl)pyridinium et ses sels pharmaceutiquement acceptables,

(h) le chlorure du 1-(2-phénylamino-2-oxoéthyl)-3-(phénylsulfonylhydrazinocarbonyl)pyridinium et ses sels pharmaceutiquement acceptables,

(i) le chlorure du 1-(2-phénylamino-2-oxoéthyl)-3-((4-méthylphényl)sulfonylhydrazinocarbonyl)pyridinium et ses sels pharmaceutiquement acceptables,

(j) le bromure du 1-(2-phényl-2-oxoéthyl)-3-(2-(benzoyloxy)éthyloxycarbonyl)pyridinium et ses sels pharmaceutiquement acceptables,

(k) le bromure du 1-(2-thién-2'-yl-2-oxoéthyl)-3-(phénylcarbonylhydrazinocarbonyl)pyridinium et ses sels pharmaceutiquement acceptables,

(l) le bromure du 1-(2-éthoxy-2-oxoéthyl)-3-((phénylméthyl)sulfonylhydrazinocarbonyl)pyridinium et ses sels pharmaceutiquement acceptables,

(m) le bromure du 1-(2-phényl-2-oxoéthyl)-3-((phénylméthyl)sulfonylhydrazinocarbonyl)pyridinium et ses sels pharmaceutiquement acceptables,

(n) le dibromure de la N,N'-bis[3-carbonyl-1-(2-furan-2'-yl-2-oxoéthyl)pyridinium]hydrazine,

(o) le bromure du 1-(2',4'-dichlorophényl-2-oxoéthyl)-3-(2-méthoxyéthylaminocarbonyl)pyridinium,

(p) le chlorure du 1-(2-thién-2'-yl-2-oxoéthyl)-3-((2-méthoxyéthyl)amincarbonyl)-5-bromopyridinium,

(q) le chlorure du 1-(2-thién-2'-yl-2-oxoéthyl)-3-(méthansulfonylhydrazinocarbonyl)pyridinium,

(r) le chlorure du 1-(2-thién-2'-yl-2-oxoéthyl)-3-(2-(2-chloro-3-pyridoylhydrazinocarbonyl)pyridinium,

(s) le chlorure du 1-(2-cyclopropylamino-2-oxoéthyl)-3-(2-méthoxyéthylaminocarbonyl)pyridinium,

(t) le chlorure du 1-(2-isopropylamino-2-oxoéthyl)-3-(2-méthylsulfonylhydrazinocarbonyl)pyridinium,

(u) le bromure du 1-(2-phénylamino-2-oxoéthyl)-3-((2-(1-oxo-3-cyclohexyl)propyl)hydrazinocarbonyl)-pyridinium,

(v) le bromure du 1-(2-thién-2'-yl-2-oxoéthyl)-3-[2-(benzoyloxy)éthylaminocarbonyl]pyridinium,

(w) le chlorure du 1-(4-éthoxy-2,4-dioxobutyl)-3-(2-(benzoxyloxy)-éthylaminocarbonyl)pyridinium,

(x) le chlorure du 1-(2-thién-2'-yl-2-oxoéthyl)-3-[1-oxo-1-(2-méthoxycarbonyl)pyridyl]hydrazinopyridinium,

(y) le dichlorure de la 1-[1-(2-thién-2'-yl-2-oxoéthyl)-5-aminocarbonyl-3-carbonylpyridinium]-2-[1-(2-thién-2'-yl-2-oxoéthyl)-3-carbonylpyridinium]hydrazine,

(z) le bromure du 1-(2-thién-2'-yl-2-oxoéthyl)-3-(trifluorométhanesulfonylhydrazinocarbonyl)pyridinium,

(aa) le dichlorure de la 1-[1-(2-thién-2'-yl-2-oxoéthyl)-6-méthyl-3-carbonylpyridinium]-2-[1-(2-thién-2'-yl-2-oxoéthyl)-3-carbonylpyridinium]hydrazine,

(ab) le dichlorure de la N,N'-bis[3-carbonyl-1-(2-(5-méthylthién-2-yl)-2-oxoéthyl)pyridinium]hydrazine,

(ac) le dichlorure de la N,N'-bis[3-carbonyl-1-(2-(5-chlorothién-2-yl)-2-oxoéthyl)pyridinium]hydrazine,

(ad) le bromure du 1-(2-thién-2'-yl-2-oxoéthyl)-3-(méthanesulfonylhydrazinocarbonyl)-6-méthylpyridinium,

(ae) le dichlorure de la N,N'-bis[3-carbonyl-1-(2-(4-nitrothién-2-yl)-2-oxoéthyl)pyridinium]hydrazine,

(af) le chlorure du 1-(2-phénylamin-2-oxoéthyl)-3-(phénylhydrazinocarbonyl)pyridinium,

(ag) le bromure du 1-(2-(4-nitrothién-2-yl)-2-oxoéthyl)-3-(méthanesulfonylhydrazinacarbonyl)pyridinium,

(ah) le chlorure du 1-(2-(5-nitrothién-2-yl)-2-oxoéthyl)-3-(méthanesulfonylhydrazinocarbonyl)-pyridinium,

(ai) le bromure du 1-(2-(5-chlorothién-2-yl)-2-oxoéthyl)-3-(méthanesulfonylhydrazinocarbonyl)-pyridinium,

(aj) le bromure du 1-(2-thién-2'-yl-2-oxoéthyl)-3-(éthoxycarbonylhydrazinocarbonyl)pyridinium,

(ak) le bromure du 1-(2-thién-2'-yl-2-oxoéthyl)-3-(isopropylsulfonylhydrazinocarbonyl)pyridinium,

(al) le bromure du 1-(2-thién-2'-yl-2-oxoéthyl)-3-(méthanesulfonylhydrazinocarbonyl)-5-bromopyridinium,

(am) le chlorure du 1-(2-(2-éthoxycarbonylpyrrolidin-1-yl)-2-oxoéthyl)-3-(méthanesulfonylhydrazinocarbonyl)-pyridinium,

(an) le chlorure du 1-(2-(5-méthylthién-2-yl)-2-oxoéthyl)-3-(méthanesulfonylhydrazinocarbonyl)-pyridinium,

(ao) le chlorure du 1-(2-(4-carbéthoxythiazolidin-3-yl)-2-oxoéthyl)-3-(méthanesulfonylhydrazinocarbonyl)-pyridinium,

(ap) le chlorure du 1-(2-(4-benzylpipéridin-1-yl)-2-oxoéthyl)-3-(méthanesulfonylhydrazinocarbonyl)-pyridinium,

(aq) le dichlorure de la N,N'-bis[3-carbonyl-2-(2-(2-éthoxycarbonylpyrrolidin-1-yl)-oxoéthyl)pyridinium]-hydrazine,

(ar) le chlorure du 1-(2-phénylamino-2-oxoéthyl)-4-[2-(benzoyloxy)éthylaminocarbonyl]pyridinium,

(as) le bromure du 1-(2-thién-2'-yl-2-oxoéthyl)-3-(phénylhydrazinocarbonyl)pyridinium,

(at) le bromure du 1-(2-thién-2'-yl-2-oxoéthyl)-3-(p-méthoxyphénylsulfonylhydrazinocarbonyl)pyridinium,

(au) le bromure du 1-(2-éthoxy-2-oxoéthyl)-3-(phénylaminocarbonylhydrazinocarbonyl)pyridinium,

(av) le bromure du 1-(2-éthoxy-2-oxoéthyl)-3-(p-toluènesulfonylhydrazinocarbonyl)pyridinium,

(aw) le bromure du 1-(2-phényl-2-oxoéthyl)-3-(phénylaminocarbonylhydrazinocarbonyl)pyridinium,

(ax) le chlorure du 1-(2-phénylamino-2-oxoéthyl)-3-benzylsulfonylhydrazinocarbonyl)pyridinium,

(ay) le bromure du 1-(2-phényl-2-oxoéthyl)-4-(méthanesulfonylhydrazinocarbonyl)pyridinium,

(az) le bromure du 1-(2-phényl-2-oxoéthyl)-3-(phénylhydrazinocarbonyl)pyridinium,

(ba) le bromure du 1-(2-éthoxy-2-oxoéthyl)-4-[2-(benzoyloxy)éthylaminocarbonyl]pyridinium,

(bb) le bromure du 1-(2-éthoxy-2-oxodithyl)-3-(phénylhydrazinocarbonyl)pyridinium,

(bc) le bromure du 1-(2-phényl-2-oxoéthyl)-3-(p-méthoxyphénylsulfonylhydrazinocarbonyl)pyridinium,

(bd) le bromure du 1-(2-phényl-2-oxoéthyl)-4-[2-(benzoyloxy)éthylaminocarbonyl]pyridinium et

(be) le bromure du 1-(2-éthoxy-2-oxoéthyl)-4-(p-méthanesulfonylhydrazinocarbonyl)pyridinium.